(19) Europäisches Patentamt
European Patent Office
Office européen des brevets

(11) Veröffentlichungsnummer : **0 031 304**
**B1**

(12) # EUROPÄISCHE PATENTSCHRIFT

(45) Veröffentlichungstag der Patentschrift : **13.06.84**

(21) Anmeldenummer : **80810396.4**

(22) Anmeldetag : **15.12.80**

(51) Int. Cl.$^3$ : **C 07 D211/58**, C 07 D211/46, C 07 D401/12, C 08 K 5/34

(54) **Piperidinderivate als Stabilisatoren für synthetische Polymere.**

(30) Priorität : **21.12.79 IT 2832479**

(43) Veröffentlichungstag der Anmeldung : **01.07.81 Patentblatt 81/26**

(45) Bekanntmachung des Hinweises auf die Patenterteilung : **13.06.84 Patentblatt 84/24**

(84) Benannte Vertragsstaaten : **CH DE FR GB IT LI**

(56) Entgegenhaltungen :
**DE-A- 2 126 954**
**FR-A- 2 183 293**
**FR-A- 2 183 294**
**FR-A- 2 256 159**
**FR-A- 2 333 794**
**FR-A- 2 349 574**
**FR-A- 2 357 561**
**FR-A- 2 405 247**
**FR-A- 2 409 985**
**US-A- 4 102 858**
**Die Akte enthält technische Angaben, die nach dem Eingang der Anmeldung eingereicht wurden und die nicht in dieser Patentschrift enthalten sind.**

(73) Patentinhaber : **Chimosa Chimica Organica S.p.A.**
**Via Pila 6/3**
**I-40044 Pontecchio Marconi (Bologna) (IT)**

(72) Erfinder : **Cantatore, Giuseppe, Dr.**
**Via Lido, 112**
**Casalecchio di Reno (Bologna) (IT)**

(74) Vertreter : **Stamm, Otto et al**
**Patentabteilung der CIBA-GEIGY AG Postfach**
**CH-4002 Basel (CH)**

**EP 0 031 304 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neuartige, als Licht-, Wärme- und Oxydationsstabilisatoren für synthetische Polymere verwendbare Piperidinverbindungen sowie Verfahren zu deren Herstellung und mit diesen Verbindungen stabilisierte Polymere.

Aus DE-A-2 126 954 und FR-A-2 183 294 sind Polyalkylpiperidincarbamate als Lichtstabilisatoren bekannt. Diese Verbindungen sind dadurch charakterisiert, dass an der Polyalkylpiperidin-4-oxycarbonylgruppe ein durch ein oder zwei Kohlenwasserstoffreste substituiertes Amin verknüpft ist.

Es wurde nun gefunden, dass Polyalkylpiperidincarbamate, bei denen über den Aminrest direkt oder indirekt eine weitere Polyalkylpiperidingruppe oder bei denen an der Polyalkylpiperidin-4-oxycarbonylgruppe ein mindestens ein Stickstoffatom enthaltender heterocyclischer Rest verknüpft ist, überraschenderweise eine bedeutend stärkere Wirksamkeit gewährleisten.

Im einzelnen betrifft die Erfindung neuartige Piperidinester der Carbaminsäure der Formel I

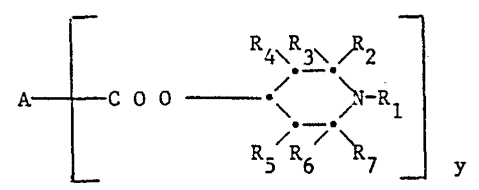

(I)

bzw. deren Säureadditionssalze, worin $R_1$ Wasserstoff, $C_1$-$C_{12}$-Alkyl, —$CH_2CN$, $C_3$-$C_{12}$-Alkenyl oder -Alkinyl, gegebenenfalls durch 1 bis 3 $C_1$-$C_4$-Alkylreste substituiertes Benzyl, gegebenenfalls durch 1 bis 3 $C_1$-$C_4$-Alkylreste substituiertes Hydroxybenzyl, einen Rest —$COR_8$, —$COOR_8$, —$CH_2COOR_8$ oder —$CONR_8R_9$, worin $R_8$ und $R_9$ gleich oder verschieden sein können und für lineares oder verzweigtes $C_1$-$C_{12}$-Alkyl, $C_3$-$C_{12}$-Alkenyl, $C_5$-$C_{12}$-Cycloalkyl, gegebenenfalls durch 1 bis 3 $C_1$-$C_8$-Alkylreste substituiertes Phenyl, gegebenenfalls durch 1 bis 3 $C_1$-$C_8$-Alkylreste substituiertes Hydroxyphenyl, $C_7$-$C_{12}$-Aralkyl, 2, 2, 6, 6-Tetramethyl-piperidin-4-yl oder 1, 2, 2, 6, 6-Pentamethyl-piperidin-4-yl stehen oder, falls sie an Stickstoff gebunden sind, Wasserstoff sein oder zusammen mit dem N-Atom, an das sie gebunden sind, einen Pyrrolidin-, Piperidin-, Morpholin-, Piperazin-, N-Methylpiperazin-, Homopiperazin- oder N-Methylhomopiperazinring bilden können, oder einen Rest

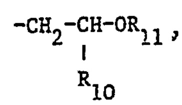

in dem $R_{10}$ für Wasserstoff, Methyl oder Phenyl und $R_{11}$ für Wasserstoff, —$R_8$, —$COR_8$ oder —$CONR_8R_9$ stehen, wobei $R_8$ und $R_9$ die obige Bedeutung haben, oder einen Rest

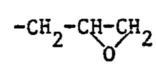

darstellt, $R_2$, $R_3$, $R_6$ und $R_7$ gleich oder verschieden sein können und Alkyl mit 1 bis 6 C-Atomen bedeuten, $R_4$ und $R_5$ gleich oder verschieden sein können und für Wasserstoff oder Alkyl mit 1 bis 6 C-Atomen stehen und y eine ganze Zahl von 1 bis 4 ist.

Mit y = 1 stellt A einen Rest,

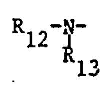

worin $R_{12}$ ein Rest der Formel II

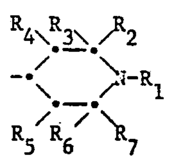

(II)

ist, worin $R_1$, $R_2$, $R_3$, $R_4$, $R_5$, $R_6$ und $R_7$ die obigen Bedeutungen haben und $R_{13}$ Wasserstoff, lineares oder verzweigtes $C_1$-$C_{20}$-Alkyl, $C_5$-$C_{12}$-Cycloalkyl, Phenyl, o-, m- und p-Toluyl, o-, m- und p-Hydroxyphenyl, 3,5-Di-t-butyl-4-hydroxyphenyl, o-, m- und p-Methoxyphenyl, o-, m- und p-Aethoxyphenyl, Benzyl, 4-Methylbenzyl, 4-t-Butylbenzyl, 4-Hydroxybenzyl, 3,5-Di-t-butyl-4-hydroxybenzyl, 4-Methoxybenzyl, 4-Aethoxybenzyl oder eine Gruppe der Formel II bedeutet, oder Pyrrolidin-1-yl, Piperidin-1-yl, Morpholin-4-yl, Piperazin-1-yl, 4-Methyl-piperazin-1-yl, Hexahydroazepin-1-yl, Homopiperazin-1-yl oder 4-Methyl-homopiperazin-1-yl dar.

Mit y = 2 stellt A einen Rest der Formel III

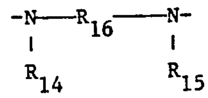 (III)

worin $R_{14}$ und $R_{15}$ gleich oder verschieden sein können und $C_5$-$C_{12}$-Cycloalkyl oder einen Rest der Formel II bedeuten und $R_{16}$ für $C_1$-$C_{20}$-Alkylen, $C_5$-$C_{12}$-Cycloalkylen, $C_6$-$C_{12}$-Arylen oder $C_7$-$C_{12}$-Aralkylen steht, oder einen Rest

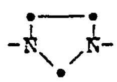 , 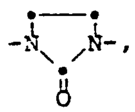 , 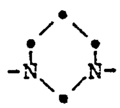 , 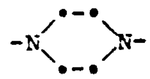 , 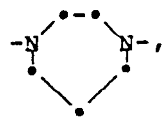 ,

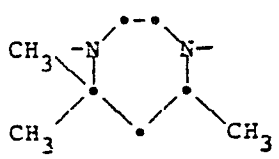 , 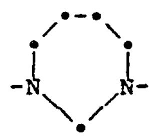 dar ;

Mit y = 3 stellt A einen Rest der Formel IV

$$R_{17}-N-(CH_2)_m-N-(CH_2)_n-N-R_{18} \quad (IV)$$

worin $R_{17}$ und $R_{18}$ gleich oder verschieden sein können und $C_1$-$C_{12}$-Alkyl, $C_5$-$C_{12}$-Cycloalkyl oder einen Rest der Formel II bedeuten sowie m und n gleich oder verschieden sein können und ganze Zahlen von 2 bis 6 sind, oder einen Rest.

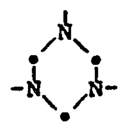 or 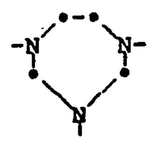

dar.

Mit y = 4 stellt A einen Rest der Formel V

$$R_{17}-N-(CH_2)_r-N-(CH_2)_s-N-(CH_2)_t-N-R_{18} \quad (V)$$

worin $R_{17}$ und $R_{18}$ die oben angegebenen Bedeutungen haben sowie r, s und t gleich oder verschieden sein können und ganze Zahlen von 2 bis 6 sind, oder einen Rest

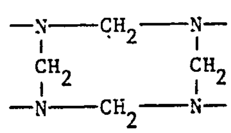

dar.

Die Bedeutungen der verschiedenen Reste speziell erläuternde Beispiele sind wie folgt :

Für $R_1$ : Wasserstoff, Methyl, Cyanmethyl, Aethyl, n-Propyl, n-Butyl, Isobutyl, n-Hexyl, n-Octyl, n-Decyl, n-Dodecyl, Allyl, 2-Butenyl, 10-Undecenyl, Propargyl, Benzyl, 4-Methylbenzyl, 4-t-Butylbenzyl, 4-Hydroxybenzyl und 3,5-Di-t-butyl-4-hydroxybenzyl.

Für $R_8$ und $R_9$ : Methyl, Aethyl, n-Propyl, n-Butyl, n-Hexyl, n-Octyl, n-Decyl, n-Dodecyl, Allyl, 2-Butenyl, 10-Undecenyl, Cyclohexyl, 2- und 4-Methylcyclohexyl, 3, 3, 5-Trimethylcyclohexyl, Phenyl, 2- und 4-Methylphenyl, 2,4- und 2,6-Dimethylphenyl, 4-t-Butylphenyl, 3,5-Di-t-butyl-4-hydroxyphenyl, Benzyl, 4-Methylbenzyl, 4-t-Butylbenzyl, 2,2,6,6-Tetramethyl-piperidin-4-yl und 1,2,2,6,6-Pentamethyl-piperidin-4-yl.

Für die Gruppe

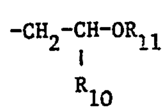

$-CH_2CH_2OH$, $-CH_2CH(OH)CH_3$, $-CH_2CH(OH)C_6H_5$, $-CH_2CH_2OC_2H_5$, $-CH_2CH_2OC_4H_9$,

$—CH_2CH_2OCOCH_3$, $—CH_2CH_2OCOC_3H_7$, $—CH_2CH_2OCON(CH_3)_2$ und $—CH_2CH_2OCON(C_2H_5)_2$.

Für $R_2$, $R_3$, $R_6$ und $R_7$: Methyl und Aethyl.

Für $R_4$ und $R_5$: Wasserstoff und Methyl.

Für $R_{12}$: 2,2,6,6-Tetramethyl-piperidin-4-yl, 1,2,2,6,6-Pentamethyl-piperidin-4-yl, 1-Allyl-2,2,6,6-tetramethyl-piperidin-4-yl, 1-Benzyl-2,2,6,6-piperidin-4-yl und 1-Acetyl-2,2,6,6-tetramethylpiperidin-4-yl.

Für $R_{13}$: Wasserstoff, Methyl, Aethyl, n-Propyl, Isopropyl, n-Butyl, Isobutyl, n-Hexyl, n-Heptyl, n-Octyl, n-Decyl, n-Dodecyl, n-Hexadecyl, n-Octadecyl, Cyclohexyl, 2- und 4-Methylcyclohexyl, 3,3,5-Trimethylcyclohexyl, Cyclooctyl, Cyclododecyl, Phenyl, o-, m- und p-Toluyl, o-, m- und p-Hydroxyphenyl, 3,5-Di-t-butyl-4-hydroxyphenyl, o-, m- und p-Methoxyphenyl, o-, m- und p-Aethoxyphenyl, Benzyl, 4-Methylbenzyl, 4-t-Butylbenzyl, 4-Hydroxybenzyl, 3,5-Di-t-butyl-4-hydroxybenzyl, 4-Methoxybenzyl, 4-Aethoxybenzyl, 2,2,6,6-Tetramethyl-piperidin-4-yl, 1,2,2,6,6-Pentamethyl-piperidin-4-yl, 1-Allyl-2,2,6,6-Tetramethyl-piperidin-4-yl, 1-Benzyl-2,2,6,6-tetramethyl-piperidin-4-yl und 1-Acetyl-2,2,6,6-tetramethyl-piperidin-4-yl.

Für $R_{14}$ und $R_{15}$: Cyclohexyl, 3,3,5-Trimethylcyclohexyl, 2,2,6,6-Tetramethyl-piperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl.

Für $R_{16}$: Methylen, Aethylen, 1,2-Propylen, Trimethylen, Tetramethylen, Pentamethylen, Hexamethylen, Dekamethylen, Dodekamethylen, Cyclohexylen, Cyclohexylen-dimethylen, o-, m- und p-Phenylen und o-, m- und p-Xylylen.

Für $R_{17}$ und $R_{18}$: Methyl, Aethyl, n-Propyl, Isopropyl, n-Butyl, 2-Butyl, Isobutyl, n-Hexyl, n-Heptyl, n-Octyl, n-Decyl, n-Dodecyl, Cyclohexyl, 3,3,5-Trimethylcyclohexyl, 2,2,6,6-Tetramethyl-piperidin-4-yl, 1,2,2,6,6-Pentamethyl-piperidin-4-yl, 1-Allyl-2,2,6,6-tetramethylpiperidin-4-yl, 1-Benzyl-2,2,6,6-tetramethyl-piperidin-4-yl und 1-Acetyl-2,2,6,6-tetramethyl-piperidin-4-yl.

m, n, r, s und t sind vorzugsweise 2, 3 oder 6.

Bevorzugt werden Verbindungen der Formel I, die dadurch gekennzeichnet sind, dass $R_1$ für Wasserstoff, $C_1$-$C_6$-Alkyl, $C_3$-$C_6$-Alkenyl, Benzyl oder $C_1$-$C_6$-Acyl, $R_2$ und $R_6$ für Methyl, $R_4$ für Wasserstoff oder Methyl, $R_5$ für Wasserstoff, $R_3$ und $R_7$ für Methyl oder Aethyl, $R_{12}$ für einen Rest der Formel II, $R_{13}$ für Wasserstoff, $C_1$-$C_{12}$-Alkyl, $C_6$-$C_{12}$-Cycloalkyl, 2,2,6,6-Tetramethylpiperidin-4-yl oder 1,2,2,6,6-Pentamethylpiperidin-4-yl, $R_{14}$ und $R_{15}$ für $C_6$-$C_{12}$-Cycloalkyl, 2,2,6,6-Tetramethylpiperidin-4-yl oder 1,2,2,6,6-Pentamethylpiperidin-4-yl, $R_{16}$ für $C_1$-$C_6$-Alkylen, $R_{17}$ und $R_{18}$ für $C_1$-$C_8$-Alkyl, $C_6$-$C_{12}$-Cycloalkyl, 2,2,6,6-Tetramethylpiperidin-4-yl oder 1,2,2,6,6-Pentamethylpiperidin-4-yl, A, falls es einen heterocyclischen Rest darstellt, für Piperidin-1-yl, Morpholin-4-yl, Piperazin-1,4-diyl oder Hexahydro-1,3,5-triazin-1,3,5-triyl sowie m, n, r, s und t für 2 oder 3 stehen.

Besonders bevorzugt werden Verbindungen der Formel I, die dadurch gekennzeichnet sind, dass $R_1$ für Wasserstoff, Methyl, Allyl, Benzyl oder Acetyl, $R_2$, $R_3$, $R_6$ und $R_7$ für Methyl, $R_4$ und $R_5$ für Wasserstoff, $R_{12}$ für 2,2,6,6-Tetramethylpiperidin-4-yl oder 1,2,2,6,6-Pentamethylpiperidin-4-yl, $R_{13}$ für Wasserstoff, $C_4$-$C_8$-Alkyl, Cyclohexyl, 2,2,6,6-Tetramethylpiperidin-4-yl oder 1,2,2,6,6-Pentamethylpiperidin-4-yl, $R_{14}$, $R_{15}$, $R_{17}$ und $R_{18}$ für Cyclohexyl, 2,2,6,6-Tetramethylpiperidin-4-yl oder 1,2,2,6,6-Pentamethylpiperidin-4-yl und $R_{16}$ für $C_2$-$C_6$-Alkylen stehen.

In den Rahmen der Erfindung fallen auch die Salze von Verbindungen der Formel I, welche durch Anlagerung von Säuren an die basischen Piperidingruppen gebildet werden. Solche Säuren sind anorganische Säuren, wie Schwefelsäure, Salzsäure oder Phosphorsäure, oder organische Säuren wie Carbonsäuren, Sulfonsäuren, Phosphonsäuren oder Phosphinsäuren. Ist $R_1$ eine Acylgruppe wie $—COR_8$, $—COOR_8$ oder $—CONR_8R_9$, ist die Piperidingruppe nicht basisch, und es bilden sich keine stabilen Salze.

Die erfindungsgemässen, neuartigen Piperidinverbindungen lassen sich herstellen durch

a) Umesterung eines Piperidinols der Formel VI

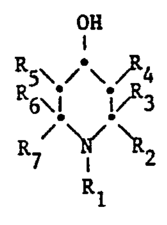

(VI)

mit einem Ester der Formel VII

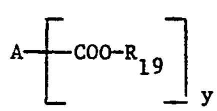

(VII)

worin $R_{19}$ für $C_1$-$C_4$-Alkyl oder Phenyl, vorzugsweise Methyl oder Aethyl, steht, in Gegenwart eines Umesterungskatalysators, beispielsweise eines Alkalialkoholats wie Natrium-methylat oder Kalium-tert.-butylat, $LiNH_2$, eines Aluminiumalkoholats wie Aluminiumisopropylat, eines Tetraalkyltitanats wie

Tetrabutyltitanat, Dibutyl-zinnoxyd oder $Sb_2O_3$, gegebenenfalls in Gegenwart eines Lösungsmittels und bei einer Temperatur zwischen 100 und 250 °C und einem Druck zwischen 0,1 und 760 Torr, oder

b) Umsetzung eines Chlorkohlensäureesters der Formel VIII

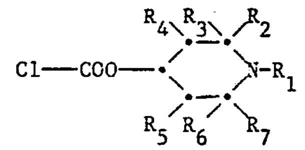

(VIII)

oder dessen Hydrochlorids mit einem Amin der Formel IX

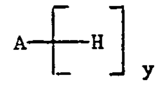

(IX)

in Gegenwart einer anorganischen Base wie Natrium- oder Kalium-bicarbonat, -carbonat oder -hydroxyd oder einer organischen Base wie Pyridin, Triäthylamin oder Tributylamin, gegebenenfalls in Gegenwart eines Lösungsmittels, bei einer Temperatur zwischen −30 und 100 °C, vorzugsweise zwischen − 10 und 60 °C.

Zur besseren Veranschaulichung der Erfindung sind unten einige Herstellungsbeispiele für Verbindungen der Formel I angegeben ; diese Beispiele dienen ausschliesslich der Erläuterung und bedeuten keinerlei Einschränkung der Erfindung.

Beispiel 1

Man gibt eine Lösung von 1 g Natrium in 30 ml Methanol zu 56,8 g (0,2 Mol) N-Butyl-N-äthoxycarbonyl-2,2,6,6-tetramethyl-4-piperidylamin und 34,54 g (0,22 Mol) 2,2,6,6-Tetramethyl-4-hydroxypiperidin.

Man erhitzt auf 180 °C unter Entfernung des bei der Reaktion freigesetzten Methanols und Aethanols ; danach hält man das Reaktionsgemisch 6 Stunden bei 180-190 °C. Anschliessend giesst man in Wasser und filtriert den erhaltenen Niederschlag ab, wäscht mit Wasser, trocknet und kristallisiert aus Methanolum. Dabei erhält man die Verbindung der Formel :

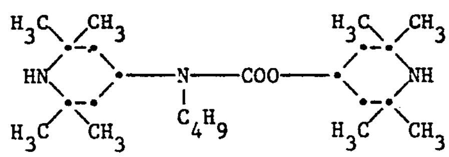

die bei 80-81 °C schmilzt.

Analyse für $C_{23}H_{45}N_3O_2$

berechnet : C 69,83 % ; H 11,46 % ; N 10,62 %.

gefunden : C 69,10 % ; H 11,34 % ; N 10,38 %.

Das N-Butyl-N-äthoxycarbonyl-2,2,6,6-tetramethyl-4-piperidylamin wird durch Umsetzung von N-Butyl-2,2,6,6-tetramethyl-4-piperidylamin mit Chlorkohlensäureäthylester bei 15-25 °C in Toluol in Gegenwart einer wässrigen Lösung von $K_2CO_3$ hergestellt. Das erhaltene 99,2 % reine Produkt wird direkt ohne jegliche Reinigung weiterverwendet.

Beispiel 2

Man gibt 68 g (0,2 Mol) N-Octyl-N-äthoxy-carbonyl-2,2,6,6-tetramethyl-4-piperidylamin und 34,54 g (0,22 Mol) 2,2,6,6-Tetramethyl-4-hydroxypiperidin zu einer Lösung von 1 g Natrium in einem Gemisch aus 100 ml Xylol und 30 ml Methanol und erhitzt den Ansatz auf 140 °C, unter Entfernung des bei der Reaktion freigesetzten Methanols und Aethanols. Nach 1 Stunde bei 140 °C steigert man die Temperatur auf 180 °C, wobei das Xylol abdestilliert, und hält 6 Stunden bei 180-190 °C. Nach Abkühlung versetzt man das Reaktionsgemisch mit 200 ml Xylol, filtriert den Ansatz zwecks Entfernung des Katalysators, wäscht das Filtrat mehrfach mit Wasser bis auf pH 8 im Waschwasser und dampft dann zur Trockne ein. Der dabei erhaltene ölige Rückstand wird in Hexan gelöst und die Lösung auf − 10 °C abgekühlt ; der entstandene kristalline Niederschlag wird abfiltriert und aus Hexan umkristallisiert.

Dabei erhält man die bei 75-6 °C schmelzende Verbindung der Formel :

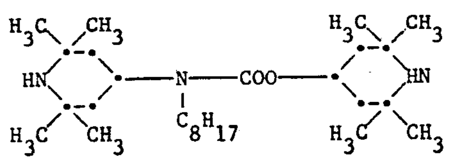

Analyse für $C_{27}H_{53}N_3O_2$
berechnet: C 71,79 %; H 11,83 %; N 9,30 %;
gefunden: C 71,71 %; H 12,01 %; N 9,27 %.

Das N-Octyl-N-äthoxycarbonyl-2,2,6,6-tetramethyl-4-piperidylamin wird durch Umsetzung von N-Octyl-2,2,6,6-tetramethyl-4-piperidylamin mit Chlorkohlensäureäthylester bei 10-25 °C in Toluol in Gegenwart einer wässrigen Lösung von $K_2CO_3$ hergestellt. Das erhaltene, 99,5 % reine Produkt wird direkt ohne jegliche Reinigung weiterverwendet.

## Beispiel 3

Die (nach Umkristallisieren aus Octan) bei 148-9 °C schmelzende Verbindung der Formel

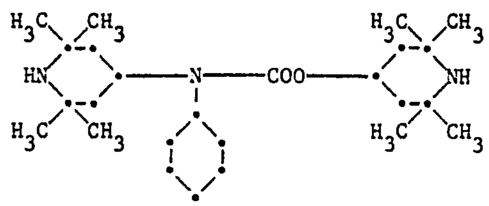

mit folgenden Analysendaten
berechnet für $C_{25}H_{47}N_3O_2$: C 71,21 %; H 11,23 %; N 9,96 %;
gefunden: C 70,24 %; H 11,19 %; N 9,89 %;
wird gemäss dem in Beispiel 2 beschriebenen Verfahren aus N-Cyclohexyl-N-äthoxycarbonyl-2,2,6,6-tetramethyl-4-piperidylamin hergestellt.

Die zuletzt erwähnte Verbindung wird durch Umsetzung von N-Cyclohexyl-2,2,6,6-tetramethyl-4-piperidylamin mit Chlorkohlensäureäthylester in Toluol in Gegenwart wässriger NaOH erhalten. Das entstandene Produkt besitzt eine Reinheit von 95 % und wird nach um Kristallisieren aus Hexan eingesetzt.

## Beispiel 4

Die (nach Umkristallisieren aus Aceton) bei 71-2 °C schmelzende Verbindung der Formel

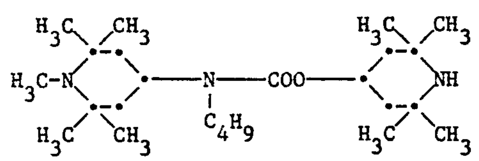

mit folgenden Analysendaten
berechnet für $C_{24}H_{47}N_3O_2$: C 70,37 %; H 11,56 %; N 10,26 %;
gefunden: C 70,17 %; H 11,46 %; N 10,10 %;
wird gemäss dem in Beispiel 2 beschriebenen Verfahren hergestellt, wobei man von N-Butyl-N-äthoxycarbonyl-1,2,2,6,6-pentamethyl-4-piperidylamin ausgeht, das seinerseits durch Umsetzung von N-Butyl-1,2,2,6,6-pentamethyl-4-piperidylamin mit Chlorkohlensäureäthylester erhalten wird.

Das entstandene Produkt mit einer Reinheit von 99,3 % wird direkt eingesetzt.

## Beispiel 5

Man gibt 99,2 g (0,2 Mol) N,N'-Bis-(äthoxycarbonyl)-N,N'-bis-(2,2,6,6-tetramethyl-piperidin-4-yl)-trimethylendiamin und 69,1 g (0,44 Mol) 2,2,6,6-Tetramethyl-4-hydroxypiperidin zu einer Lösung von 2 g Natrium in einem Gemisch aus 100 ml Xylol und 50 ml Methanol und erhitzt den Ansatz auf 140 °C, unter Entfernung des bei der Reaktion freigesetzten Methanols und Aethanols und eines Teils des Xylols, und hält 1 Stunde bei dieser Temperatur. Nach Abdestillieren des Lösungsmittels steigert man die Temperatur dann auf 180 °C und hält 6 Stunden bei 180-190 °C. Zuletzt erhitzt man das Gemisch 30 Minuten bei 160-180 °C unter vermindertem Druck (etwa 100 Torr). Man löst das erhaltene Reaktionsgemisch in 300 ml Xylol, filtriert und wäscht das Filtrat mehrfach mit Wasser bis auf pH 8 im Waschwasser und dampft zur Trockne ein.

Man erhält die bei 51-52 °C schmelzende Verbindung der Formel:

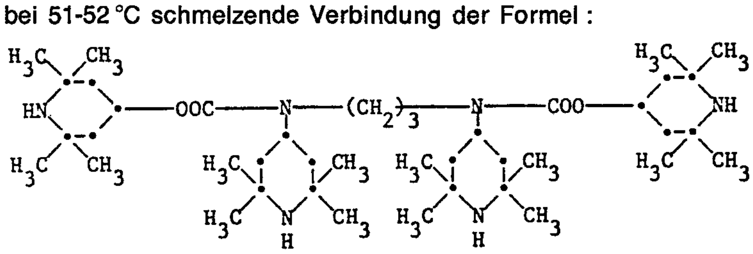

Analyse für $C_{41}H_{78}N_6O_4$
berechnet : C 68,48 % ; H 10,93 % ; N 11,69 % ;
gefunden : C 68,10 % ; H 11,02 % ; N 11,44 %.

Das N,N'-Bis-(äthoxycarbonyl)-N,N'-bis-(2,2,6,6-tetramethyl-piperidin-4-yl)-trimethylendiamin wird durch Umsetzung von N,N'-Bis-(2,2,6,6-tetramethyl-piperidin-4-yl)-trimethylendiamin mit Chlorkohlensäureäthylester in Toluol in Gegenwart wässriger NaOH erhalten. Das entstandene Produkt besitzt eine Reinheit von 99 % und wird direkt ohne jegliche Reinigung weiterverwendet.

## Beispiel 6

Man gibt eine Lösung von 2 g Natrium in 50 ml Methanol zu 107,6 g (0,2 Mol) N,N'-Bis-(äthoxycarbonyl)-N,N'-bis-(2,2,6,6-tetramethyl-piperidin-4-yl)-hexamethylendiamin und 69,1 g (0,44 Mol) 2,2,6,6-Tetramethyl-4-hydroxypiperidin. Man erhitzt auf 180 °C unter Entfernung des bei der Reaktion gebildeten Methanols und Aethanols und hält dann 8 Stunden bei 180 °C. Zuletzt erhitzt man 30 Minuten bei 160-180 °C unter vermindertem Druck (etwa 100 Torr). Nach Abkühlung auf 80 °C löst man das Reaktionsgemisch in 300 ml Xylol, filtriert und dampft das Filtrat zur Trockne ein.

Der erhaltene Rückstand wird mehrfach mit Wasser bis auf pH 8 im Waschwasser gewaschen, getrocknet und aus Hexan umkristallisiert.

Man erhält die bei 148-9 °C schmelzende Verbindung der Formel

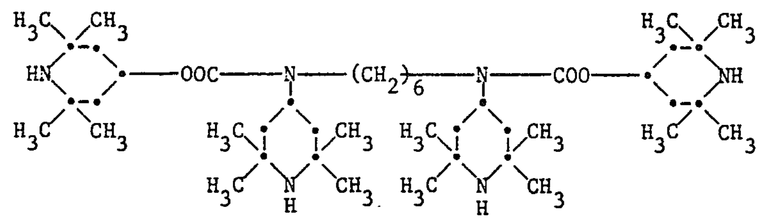

Analyse für $C_{44}H_{84}N_6O_4$
berechnet : C 69,43 % ; H 11,12 % ; N 11,04 % ;
gefunden : C 69,18 % ; H 11,13 % ; N 10,71 %.

Das N,N'-Bis-(äthoxycarbonyl)-N,N'-bis-(2,2,6,6-tetramethyl-piperidin-4-yl)-hexamethylendiamin wird durch Umsetzung von N,N'-Bis-(2,2,6,6-tetramethyl-piperidin-4-yl)-hexamethylendiamin mit Chlorkohlensäureäthylester in Toluol in Gegenwart wässriger NaOH hergestellt und aus Octan umkristallisiert.

## Beispiel 7

Die (nach Umkristallisieren aus Octan) bei 183-5 °C schmelzende Verbindung der Formel

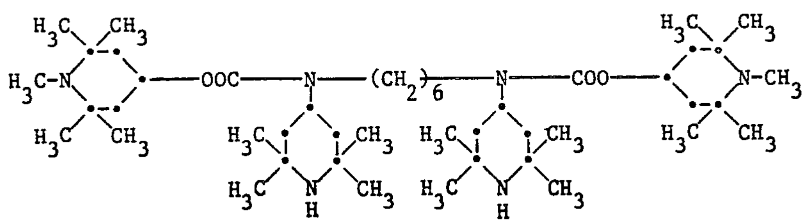

mit folgenden Analysendaten :
berechnet für $C_{46}H_{88}N_6O_4$ : C 70,00 % ; H 11,24 % ; N 10,65 % ;
gefunden : C 68,77 % ; H 11,03 % ; N 10,38 % ;
wird gemäss dem in Beispiel 6 beschriebenen Verfahren hergestellt, wobei man von N,N'-Bis-(äthoxycarbonyl)-N,N'-bis-(2,2,6,6-tetramethyl-piperidin-4-yl)-hexamethylendiamin und 1,2,2,6,6-Pentamethyl-4-hydroxypiperidin ausgeht.

## Beispiel 8

Man gibt 92 g (0,4 Mol) N,N'-Bis-(äthoxycarbonyl)-piperazin [gemäss J. ORG. CHEM. 25, 1874 (1960) hergestellt] und 131,9 g (0,84 Mol) 2,2,6,6-Tetramethyl-4-hydroxypiperidin zu einer Lösung von 2 g Natrium in einem Gemisch aus 200 ml Xylol und 40 ml Methanol.

Im Verlauf von 1 Stunde erhitzt man auf 180 °C, unter Entfernung flüchtiger Komponenten, und hält 6 Stunden bei 180-190 °C.

Nach Abkühlung auf Raumtemperatur versetzt man mit 400 ml Chloroform, rührt 30 Minuten und filtriert. Das Filtrat wird zur Trockne eingedampft und der erhaltene Rückstand zweimal aus Xylol umkristallisiert.

Dabei erhält man die bei 208-9 °C schmelzende Verbindung der Formel :

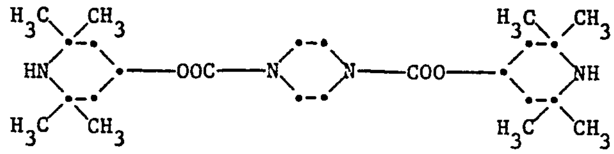

Analyse für $C_{24}H_{44}N_4O_4$
berechnet : C 63,68 % ; H 9,80 % ; N 12,38 % ;
gefunden : C 63,24 % ; H 9,65 % ; N 12,39 %.

Beispiel 9

Man gibt 60,6 g (0,2 Mol) 1,3,5-Tris-(äthoxycarbonyl)-hexahydro-1,3,5-triazin [gemäss J. Heterocycl. Chem. 1974, 11(6), 937 hergestellt] und 103,6 g (0,66 Mol) 2,2,6,6-Tetramethyl-4-hydroxypiperidin zu einer Lösung von 2 g Natrium in einem Gemisch aus 100 ml Xylol und 30 ml Methanol.

Man erhitzt auf 180 °C, unter Entfernung flüchtiger Produkte, und hält 6 Stunden bei 180-190 °C ; zuletzt erhitzt man 1 Stunde bei 150-160 °C unter vermindertem Druck (etwa 100 Torr). Das erkaltete Gemisch versetzt man mit 300 ml Xylol und filtriert den Ansatz zur Entfernung unlöslicher Stoffe. Das Filtrat wird bei Raumtemperatur 30 Minuten mit 50 ml Wasser verrührt ; es bildet sich ein kristalliner Niederschlag, der abfiltriert, mit Wasser gewaschen, getrocknet und aus Octan umkristallisiert wird.

Dabei erhält man die bei 180-181 °C schmelzende Verbindung der Formel :

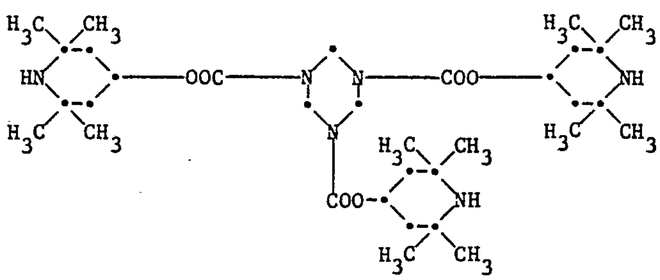

Analyse für $C_{33}H_{60}N_6O_6$
berechnet : C 62,24 % ; H 9,49 % ; N 13,20 % ;
gefunden : C 61,41 % ; H 9,26 % ; N 12,89 %.

Beispiel 10

Man gibt 60,6 g (0,2 Mol) 1,3,5-Tris-(äthoxycarbonyl)-hexahydro-1,3,5-triazin und 112,9 g (0,66 Mol) 1,2,2,6,6-Pentamethyl-4-hydroxypiperidin zu einer Lösung von 2 g Natrium in einem Gemisch aus 100 ml Xylol und 30 ml Methanol, erhitzt den Ansatz auf 180 °C, unter Entfernung flüchtiger Produkte, und hält 6 Stunden bei 180-190 °C.

Nach Abkühlung versetzt man mit 300 ml Xylol und filtriert das Gemisch ; das Filtrat wird mehrfach mit Wasser bis auf neutralen pH gewaschen und dann zur Trockne eingedampft.

Der erhaltene Rückstand wird aus Aceton umkristallisiert.

Dabei erhält man die bei 133-4 °C schmelzende Verbindung der Formel

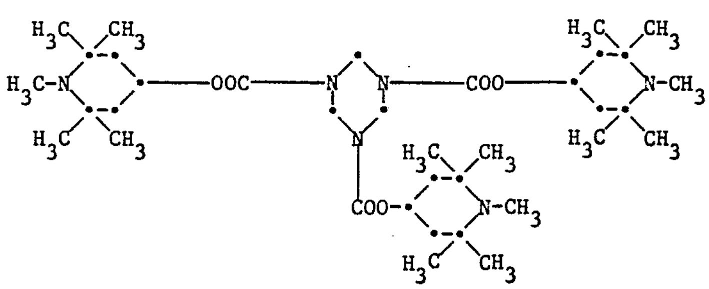

Analyse für $C_{36}H_{66}N_6O_6$
berechnet : C 63,68 % ; H 9,80 % ; N 12,38 % ;
gefunden : C 63,59 % ; H 9,84 % N 12,24 %.

Beispiel 11

Die (nach Umkristallisieren aus Hexan) bei 102-3 °C schmelzende Verbindung der Formel

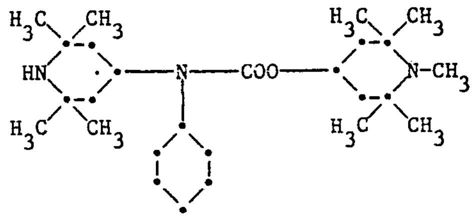

mit folgenden Analysendaten:
berechnet für $C_{26}H_{49}N_3O_6$: C 71,68 %; H 11,34; N 9,64 %;
gefunden: C 71,60 %; H 11,50 %; N 9,66 %
wird gemäss dem in Beispiel 2 beschriebenen Verfahren hergestellt, wobei man von N-Cyclohexyl-N-äthoxycarbonyl-2,2,6,6-tetramethyl-4-piperidylamin und 1,2,2,6,6-Pentamethyl-4-hydroxypiperidin ausgeht.

Beispiel 12

Man erhitzt 45,2 g (0,1 Mol) der in Beispiel 8 beschriebenen Verbindung, 40,8 g (0,4 Mol) Essigsäureanhydrid und 80,8 g (0,8 Mol) Triäthylamin 15 Stunden unter Rückfluss und filtriert dann heiss. Der erhaltene kristalline Niederschlag wird aus Toluol umkristallisiert.

Dabei erhält man die bei 179-80 °C schmelzende Verbindung der Formel

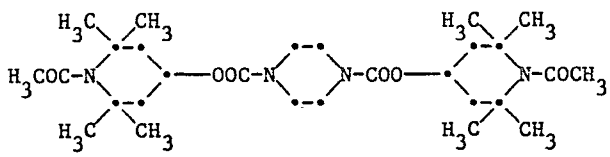

Analyse für $C_{28}H_{48}N_4O_6$:
berechnet: C 62,66 %; H 9,01 %; N 10,44 %;
gefunden: C 62,51 %; H 9,05 %; N 10,30 %.

Beispiel 13

Man erhitzt ein Gemisch aus 36,7 g (0,1 Mol) N-Aethoxycarbonyl-bis-(2,2,6,6-tetramethyl-4-piperidinyl)-amin, 17,22 g (0,11 Mol) 2,2,6,6-Tetramethyl-4-hydroxypiperidin und 1 g Natriummethylat 7 Stunden auf 200 °C sowie 2 Stunden auf 160-170 °C bei 100 Torr Vakuum. Nach Abkühlen wird das Produkt vermahlen, mit Wasser gewaschen, getrocknet und aus Aceton umkristallisiert.

Auf diese Weise erhält man die bei 187-8 °C schmelzende Verbindung der Formel

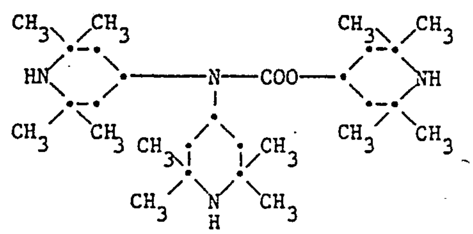

Analyse ($C_{28}H_{54}N_4O_2$):
berechnet: C 70,25 %; H 11,37 %; N 11,70 %;
gefunden: C 69,50 %; H 11,43 %; N 11,57 %.

Das N-Aethoxycarbonyl-bis-(2,2,6,6-tetramethyl-4-piperidyl)-amin wird durch Umsetzung von Bis-(2,2,6,6-tetramethyl-4-piperidyl)-amin mit Chlorkohlensäureäthylester in Chloroform bei 10-20 °C in Gegenwart wässriger NaOH hergestellt. Das bei 117 °C schmelzende Produkt mit einer Reinheit von 99,2 % kann direkt weiterverwendet werden.

Beispiel 14

Man erhitzt ein Gemisch aus 36,8 g (0,1 Mol) N,N'-Dicyclohexyl-N,N'-bis-(äthoxycarbonyl)-äthylendiamin, 34,5 g (0,22 Mol) 2,2,6,6-Tetramethyl-4-hydroxypiperidin und 1 g Natriummethylat 4 Stunden auf 190-200 °C sowie 2 Stunden auf 160-180 °C/100 Torr. Nach Abkühlen wird das Reaktionsgemisch vermahlen, mit Wasser gewaschen, getrocknet und aus Toluol umkristallisiert.

Dabei erhält man die bei 210-211 °C schmelzende Verbindung der Formel

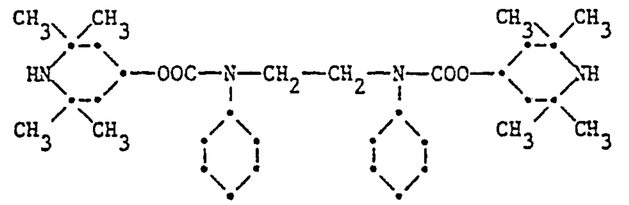

Analyse ($C_{34}H_{62}N_4O_4$) :
berechnet : C 69,11 % ; H 10,58 % ; N 9,48 % ;
gefunden : C 69,34 % ; H 10,51 % ; N 9,42 %.

Das N,N'-Dicyclohexyl-N,N'-bis-(äthoxycarbonyl)-äthylendiamin (Schmelzpunkt (108 °C) wird durch Umsetzung von N,N'-Dicyclohexyläthylendiamin mit Chlorkohlensäureäthylester in Chloroform bei 10-20 °C in Gegenwart wässriger NaOH hergestellt. Das erhaltene Produkt besitzt eine Reinheit von 99,7 % und wird direkt weiterverwendet.

Beispiel 15

Man erhitzt ein Gemisch von 48,3 g (0,1 Mol) N,N'-Dicyclohexyl-N,N',N"-tris-(äthoxycarbonyl)-diäthylentriamin, 62,8 g (0,4 Mol) 2,2,6,6-Tetramethyl-4-hydroxypiperidin und 2 g $NaOCH_3$ 8 Stunden auf 200 °C bei Normaldruck und weitere 8 Stunden im Vakuum (bis 100 Torr). Nach Abkühlen wird das Reaktionsgemisch vermahlen, mit Wasser gewaschen, getrocknet und aus Xylol umkristallisiert.

Dabei erhält man die bei 184-185 °C schmelzende Verbindung der Formel

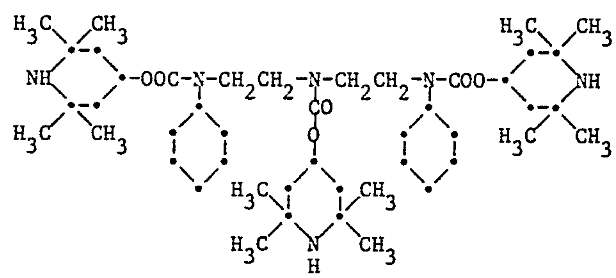

Analyse ($C_{46}H_{84}N_6O_6$) :
berechnet : C 67,61 % ; H 10,36 % ; N 10,28 % ;
gefunden : C 67,22 % ; H 10,29 % ; N 10,08 %.

Das N,N'-Dicyclohexyl-N,N',N"-tris-(äthoxycarbonyl)-diäthylentriamin (Schmelzpunkt 60-61 °C, aus Hexan) wird durch Umsetzung von N,N'-Dicyclohexyldiäthylentriamin mit Chlorkohlensäureäthylester in Toluol bei 10-20 °C in Gegenwart wässriger NaOH hergestellt.

Beispiel 16

Die in Beispiel 7 beschriebene Verbindung kann alternativ durch Umsetzung von Chlorkohlensäure-1,2,2,6,6-pentamethyl-piperidin-4-ylester-hydrochlorid mit N,N'-Bis-(2,2,6,6-tetramethylpiperidin-4-yl)-hexamethylendiamin gemäss folgender Arbeitsweise hergestellt werden :

Man gibt eine Lösung von 28,3 g (0,105 Mol) Chlorkohlensäure-1,2,2,6,6-pentamethylpiperidin-4-ylester-hydrochlorid in 70 ml Chloroform zu einer auf 0 °C gekühlten Lösung von 19,7 g (0,05 Mol) N,N'-Bis-(2,2,6,6-tetramethyl-piperidin-4-yl)-hexamethylendiamin in 50 ml Chloroform. Während der Zugabe hält man die Temperatur unter 10 °C. Das Reaktionsgemisch rührt man nach der Zugabe 1 Stunde bei einer Temperatur von etwa 10 °C und versetzt dann im Verlauf von 40 Minuten unter Rühren bei auf etwa 10 °C konstant gehaltener Temperatur mit einer Lösung von 8,8 g NaOH in 50 ml Wasser. Nach weiteren 4 Stunden Rühren bei Raumtemperatur wird die wässrige Schicht abgetrennt und die Chloroformlösung über $Na_2SO_4$ getrocknet und zur Trockne eingedampft. Den erhaltenen Rückstand kristallisiert man aus Octanum, was ein bei 186-188 °C schmelzendes Produkt liefert.

Das dabei verwendete Chlorkohlensäureester-hydrochlorid wird durch Zugabe einer Lösung von 148, 5 g Phosgen in 400 ml Acetronitril bei einer Temperatur von 0 °C zu einer Suspension von 207,5 g 1,2,2,6,6-Pentamethyl-4-hydroxypiperidin-hydrochlorid in 400 ml Acetronitril hergestellt. Nach 15 Stunden Rühren bei Raumtemperatur steigert man die Temperatur langsam auf 50 °C. Ueberschüssiges Phosgen wird

durch Einleiten eines Stickstoffstroms für 6 Stunden bei 50 °C entfernt. Schliesslich wird das Lösungsmittel im Vakuum bei 50 °C abgedampft. Als Rückstand erhält man das reine, bei 153-156 °C schmelzende Produkt.

Beispiel 17

Man erhitzt 22,8 g (0,1 Mol) N-Aethoxycarbonyl-2,2,6,6-tetramethyl-4-piperidylamin, 34,54 g (0,22 Mol) 2,2,6,6-Tetramethyl-4-hydroxypiperidin und 1 g $NaOCH_3$ 6 Stunden auf 180 °C bei Normaldruck und 1 Stunde bei etwa 100 Torr. Nach Abkühlen wird das erhaltene Gemisch vermahlen, mit Wasser gewaschen und aus Isopropanol umkristallisiert.

Die so erhaltene Verbindung der Formel

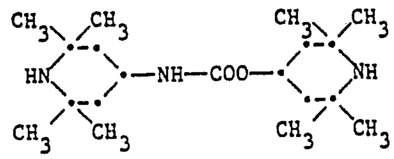

schmilzt bei 182-183 °C.

Analyse ($C_{19}H_{37}N_3O_2$) :
berechnet : C 67,21 % ; H 10,98 % ; N 12,38 % ;
gefunden : C 66,85 % ; H 10,93 % ; N 12,48 %.

Beispiel 18

Die Verbindung der Formel

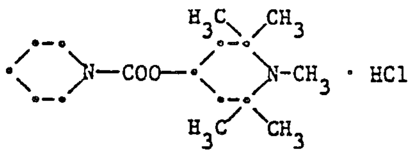

wird dadurch hergestellt, dass man 8,5 g (0,1 Mol) in 50 ml Chloroform gelöstes Piperidin bei einer Temperatur von 0 bis 10 °C mit 29,7 g (0,11 Mol) in 70 ml Chloroform gelöstem Chlorkohlensäure-1,2,2,6,6-pentamethylpiperidin-4-ylester-hydrochlorid unter Zugabe einer Lösung von 4,4 g (0,11 Mol) NaOH in 30 ml Wasser umsetzt. Die Verbindung schmilzt bei 188-189 °C (nach Umkristallisieren aus Toluol).

Analyse ($C_{16}H_{31}ClN_2O_2$) :
berechnet : C 60,27 % ; H 9,80 % ; N 8,78 % ; Cl 11,12 % ;
gefunden : C 60,46 % ; H 9,74 % ; N 8,76 % ; Cl 10,97 %.

Beispiel 19

Die bei 129-130 °C schmelzende Verbindung (aus Hexan) der Formel

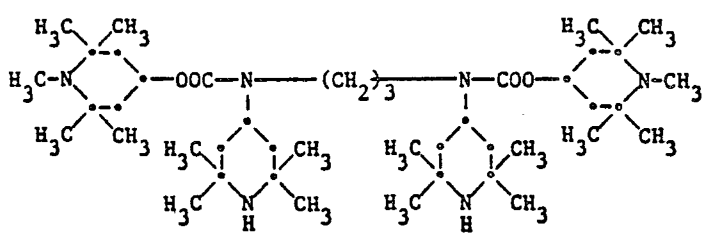

wird auf gleiche Weise wie in Beispiel 16 beschrieben aus Chlorkohlensäure-1,2,2,6,6-pentamethylpiperidin-4-ylester-hydrochlorid und N,N'-Bis-(2,2,6,6-tetramethylpiperidin-4-yl)-trimethylendiamin hergestellt.

Analyse ($C_{43}H_{82}N_6O_4$) :
berechnet : C 69,12 % ; H 11,06 % ; N 11,25 % ;
gefunden : C 69,61 % ; H 11,03 % ; N 11,20 %.

Beispiel 20

Die bei 241-242 °C schmelzende Verbindung (aus Toluol) der Formel

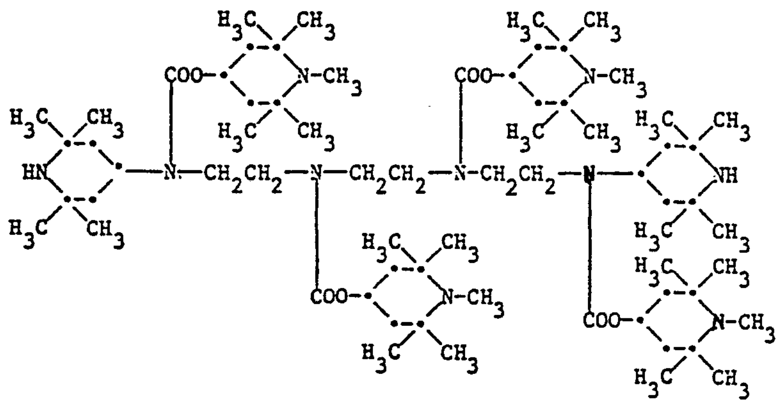

wird wie in Beispiel 16 beschrieben aus Chlorkohlensäure-1,2,2,6,6-pentamethylpiperidin-4-ylester und N,N'-Bis-(2,2,6,6-pentamethylpiperidin-4-yl)-1,8-diamino-3,6-diazaoctan hergestellt.

Analyse ($C_{68}H_{128}N_{10}O_8$) :

berechnet : C 67,29 % ; H 10,63 % ; N 11,54 % ;

gefunden : C 66,07 % ; H 10,35 % ; N 11,38 %.

Wie anfangs erwähnt, sind die Verbindungen der Formel I sehr wirksam bei der Verbesserung der Licht-, Wärme- und Oxydationsbeständigkeit von synthetischen Polymeren wie beispielsweise Polyäthylen hoher oder niedriger Dichte, Polypropylen, Aethylen/Propylencopolymeren, Aethylen/Vinylacetatcopolymeren, Polybutadien, Polyisopren, Polystyrol, Butadien/Styrolcopolymeren, Acrylnitril/Butadien/Styrolcopolymeren, Polymeren und Copolymeren von Vinylchlorid und Vinylidenchlorid, Polyoxymethylen, Poly-Polyurethanen, gesättigten und ungesättigten Polyestern, Polyamiden, Polycarbonaten, Polyacrylaten, Alkydharzen und Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen setzt man zweckmässig 0,01 bis 5 Gew.-% der Verbindungen der Formel I, bezogen auf das Gewicht der Polymeren, vorzugsweise 0,1 bis 1 %, ein.

Die Verbindungen der Formel I können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches ; das synthetische Polymer lässt sich dabei in Form von Pulver, Granulat, Lösung, Suspension oder Emulsion einsetzen. Die mit den Produkten der Formel I stabilisierten Polymeren lassen sich zur Herstellung von Formkörpern, Folien, Bändern, Endlosfäden, Lacken usw. verwenden.

Gegebenenfalls kann man den Mischungen von Verbindungen der Formel I mit den synthetischen Polymeren weitere Zusatzstoffe beigeben, wie beispielsweise Antioxydantien, UV-absorbierende Stoffe, Nickelstabilisatoren, Pigmente, Füllstoffe, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel I verwendbare Zusatzstoffe sind insbesondere :

Phenolische Antioxydantien, wie beispielsweise 2,6-Di-t-butyl-p-kresol, 4,4'-Thio-bis-(3-methyl-6-t-butyl-phenol), 1,1,3-Tris-(2-methyl-4-hydroxy-5-t-butylphenyl)-butan, Octadecyl-3-(3,5-di-t-butyl-4-hydroxyphenyl)-propionat, Pentaerythrit-tetrakis-(3,5-di-t-butyl-4-hydroxyphenyl)-propionat, Tris-(3,5-di-t-butyl-4-hydroxybenzyl)-isocyanurat und Tris-(4-t-butyl-3-hydroxy-2,6-dimethyl-benzyl)-isocyanurat ;

Sekundäre Antioxydantien, wie : Thiodipropionsäureester, beispielsweise Di-n-dodecyl-thiodipropionat und Di-n-octadecyl-thiodipropionat ; aliphatische Sulfide und Disulfide, beispielsweise Di-n-dodecylsulfid, Di-n-octadecylsulfid und Di-n-octadecyldisulfid ; aliphatische, aromatische und aliphatischaromatische Phosphite und Thiophosphite, beispielsweise Tri-n-dodecylphosphit, Tris-(nonylphenyl)-phosphit, Tri-n-dodecyltrithiophosphit, Phenyl-di-n-decylphosphit, Di-n-octadecyl-pentaerythritdiphosphit, Tris-(2,4-di-t-butylphenyl)-phosphit und Tetrakis-(2,4-di-t-butylphenyl)-4,4'-biphenylen-diphosphit ;

UV-absorbierende Stoffe, beispielsweise 2-Hydroxy-4-n-octyloxybenzophenon, 2-Hydroxy-4-n-dodecyloxybenzophenon, 2-(2-Hydroxy-3,5-di-t-butylphenyl)-5-chlorbenztriazol, 2-(2-Hydroxy-3,5-di-t-amylphenyl)-benztriazol, 2,4-Di-t-butylphenyl-3,5-di-t-butyl-4-hydroxybenzoat, Phenylsalicylat, p-t-Butylphenylsalicylat, 2,2'-Di-n-octyloxy-5,5'-di-t-butyl-oxanilid, 2-Aethoxy-5-t-butyl-2'-äthyl-oxanilid, 2-Aethoxy-2'-äthyl-oxanilid und 2-Aethoxy-2'-äthyl-5,5'-di-t-butyloxanilid ;

Polyalkylpiperidin-Lichtstabilisatoren, beispielsweise 2,2,6,6-Tetramethyl-piperidin-4-yl-benzoat, Bis-(2,2,6,6-tetramethylpiperidin-4-yl)-sebacinat, Bis-(1,2,2,6,6-pentamethylpiperidin-4-yl)-sebacinat und Bis-(1,2,2,6,6-pentamethylpiperidin-4-yl)-butyl-(3,5-di-t-butyl-4-hydroxybenzyl)-malonat ;

Lichtstabilisatoren auf Nickelbasis, beispielsweise Ni-monoäthyl-3,5-di-t-butyl-4-hydroxybenzylphosphonat, der Butylaminkomplex des Ni-2,2'-thio-bis-(4-t-octylphenolats), Ni-2,2'-thio-bis-(4-t-octylphenol-phenolat), Ni-dibutyldithiocarbamat, Ni-3,5-di-t-butyl-4-hydroxybenzoat und der Ni-Komplex des 2-Hydroxy-4-n-octyloxybenzophenons ;

Organozinnstabilisatoren, beispielsweise Dibutylzinn-maleinat, Dibutylzinn-laurat und Dioctylzinn-maleinat ;

Acrylester, beispielsweise Aethyl-α-cyan-β,β-di-phenylacrylat und Methyl-α-cyan-β-methyl-4-methoxycinnamat ;

Metallsalze höherer Fettsäuren, beispielsweise Calcium-, Barium-, Cadmium-, Zink-, Blei- und Nickelstearat sowie Calcium-, Cadmium-, Zink- und Bariumlaurat ;

Organische und anorganische Pigmente, beispielsweise Colour Index Pigment Yellow 37, Colour Index Pigment Yellow 83, Colour Index Pigment Red 144, Colour Index Pigment Red 48 : 3, Colour Index Pigment Blue 15, Colour Index Pigment Green 7, Titandioxyd und Eisenoxyd.

Die Wirksamkeit der erfindungsgemässen Produkte als Stabilisatoren wird in den nachfolgenden Beispielen veranschaulicht, wo die in den Herstellungsbeispielen erhaltenen Produkte in einer synthetischen Polymerenmasse eingesetzt werden.

Die Ergebnisse sind solchen gegenübergestellt, die unter Zusatz von handelsüblichen Lichtstabilisatoren erhalten wurden.

Beispiel 21

Je 2 g der in Tabelle 1 angeführten Verbindungen und 1 g n-Octadecyl-3-(3,5-di-t-butyl-4-hydroxyphenyl)-propionat (Antioxydans) werden mit 1 000 g Polypropylen mit einem Schmelzindex von 2,6 (Moplen T, Produkt der Società Montedison, Italien) und 1 g Calciumstearat innig vermischt.

Das erhaltene Gemisch wird dann bei einer Temperatur von 200-230 °C extrudiert und zu Granulat verarbeitet, aus dem man Bänder einer Dicke von 50 um und einer Breite von 2,5 mm herstellt. Die Arbeitsbedingungen sind :

Extrudertemperatur : 230-240 °C
Kopftemperatur : 240 °C
Streckverhältnis : 1 : 6

Die erhaltenen Bänder werden in einem Weather-Ometer 65 WR (ASTM G 27-70) bei einer Temperatur der schwarzen Tafel von 63 °C ausgesetzt. Von Zeit zu Zeit werden Proben entnommen, an denen man die verbleibende Zugfestigkeit mittels eines Tensometers mit konstanter Geschwindigkeit misst ; danach bestimmt man die zum Halbieren der ursprünglichen Zugfestigkeit erforderliche Aussetzungszeit ($T_{50}$).

Zum Vergleich wird ein Band mit Zusatz von 2 g 2-Hydroxy-4-n-octyloxybenzophenon als Lichtstabilisator unter den gleichen Bedingungen hergestellt.

Die erhaltenen Ergebnisse sind in Tabelle 1 angeführt.

Tabelle 1

| Lichtstabilisator | $T_{50}$ (Stunden) |
|---|---|
| 2-Hydroxy-4-n-octyloxybenzophenon | 300 |
| Verbindung aus Beispiel 1 | 2320 |
| Verbindung aus Beispiel 2 | 2230 |
| Verbindung aus Beispiel 3 | 2360 |
| Verbindung aus Beispiel 6 | 1980 |
| Verbindung aus Beispiel 7 | 2330 |
| Verbindung aus Beispiel 9 | 1930 |
| Verbindung aus Beispiel 10 | 1770 |
| Verbindung aus Beispiel 11 | 2180 |
| Verbindung aus Beispiel 13 | 2040 |

Beispiel 22

Je 2 g der in Tabelle 2 aufgeführten Ververbindungen werden mit 1 000 g Polyäthylen hoher Dichte und mit einem Schmelzindex von 0,40 (Hostalen GF 7660, Hoechst AG, Bundesrepublik Deutschland), 0,5 g n-Octadecyl-β-(3,5-di-t-butyl-4-hydroxyphenyl)-propionat (als Antioxydans) und 1 g Calciumstearat gründlich vermischt.

Das erhaltene Gemisch wird dann bei einer Temperatur von 190 °C extrudiert und zu Granulat zerschnitten, aus dem man durch Formpressen bei 200 °C Folien von 0,2 mm Dicke erhält. Die Folien werden in einem Weather-Ometer 65 WR mit einer Temperatur der schwarzen Tafel von 63 °C ausgesetzt, und von Zeit zu Zeit überprüft man die Zunahme des Carbonylgruppengehalts (ΔCO) unter Verwendung von nicht ausgesetzten Proben zum Ausgleich der Anfangsabsorption des Polymeren. Man berechnet die für ΔCO = 0,1 % (bei einer Wellenläge von 5,85 μm) erforderliche Aussetzungszeit (T 0,1).

Zum Vergleich stellt man unter den gleichen Bedingungen Proben

a) ohne Zusatz eines Lichtstabilisators

b) mit Zusatz von 2 g 2-Hydroxy-4-n-octyloxy-benzophenon her. Die Ergebnisse sind in Tabelle 2 angeführt.

Tabelle 2

| Lichtstabilisator | T 0,1 (Stunden) |
|---|---|
| keiner | 320 |
| 2-Hydroxy-4-n-octyloxy-benzophenon | 1000 |
| Verbindung aus Beispiel 1 | 5710 |
| Verbindung aus Beispiel 6 | 5350 |
| Verbindung aus Beispiel 7 | 5280 |
| Verbindung aus Beispiel 9 | 5050 |

**Ansprüche**

1. Verbindungen der Formel I

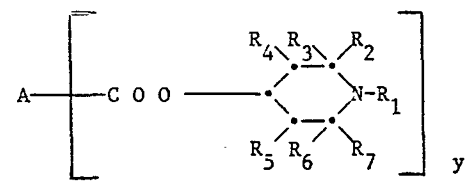

(I)

bzw. deren Säureadditionssalze, worin $R_1$ Wasserstoff, $C_1$-$C_{12}$-Alkyl, —$CH_2CN$, $C_3$-$C_{12}$-Alkenyl oder -Alkinyl, gegebenenfalls durch 1 bis 3 $C_1$-$C_4$-Alkylreste substituiertes Benzyl, gegebenenfalls durch 1 bis 3 $C_1$-$C_4$-Alkylreste substituiertes Hydroxybenzyl, einen Rest —$COR_8$, —$COOR_8$, —$CH_2COOR_8$ oder —$CONR_8R_9$, worin $R_8$ und $R_9$ gleich oder verschieden sein können und für lineares oder verzweigtes $C_1$-$C_{12}$-Alkyl, $C_3$-$C_{12}$-Alkenyl, $C_5$-$C_{12}$-Cycloalkyl, gegebenenfalls durch 1 bis 3 $C_1$-$C_8$-Alkylreste substituiertes Phenyl, gegebenenfalls durch 1 bis 3 $C_1$-$C_8$-Alkylreste substituiertes Hydroxyphenyl, $C_7$-$C_{12}$-Aralkyl, 2,2,6,6-Tetramethyl-piperadin-4-yl oder 1,2,2,6,6-Pentamethyl-piperidin-4-yl stehen oder, falls sie an Stickstoff gebunden sind, Wasserstoff sein oder zusammen mit dem N-Atom, an das sie gebunden sind, einen Pyrrolidin-, Piperidin-, Morpholin-, Piperazin, N-Methylpiperazin-, Homopiperazin- oder N-Methylhomopiperazinring bilden können, oder einen Rest

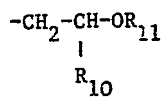

in dem $R_{10}$ für Wasserstoff, Methyl oder Phenyl und $R_{11}$ für Wasserstoff, —$R_8$, —$COR_8$ oder —$CONR_8R_9$ stehen, wobei $R_8$ und $R_9$ die obige Bedeutung haben, oder einen Rest

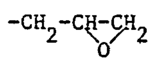

darstellt, $R_2$, $R_3$, $R_6$ und $R_7$ gleich oder verschieden sein können und Alkyl mit 1 bis 6 C-Atomen bedeuten, $R_4$ und $R_5$ gleich oder verschieden sein können und für Wasserstoff oder Alkyl mit 1 bis 6 C-Atomen stehen und y eine ganze Zahl von 1 bis 4 ist, wobei mit $y = 1$ A einen Rest

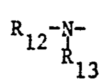

worin $R_{12}$ ein Rest der Formel II

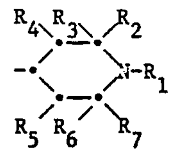

(II)

ist, worin $R_1$, $R_2$, $R_3$, $R_4$, $R_5$, $R_6$ und $R_7$ die obigen Bedeutungen haben und $R_{13}$ Wasserstoff, lineares oder verzweigtes $C_1$-$C_{20}$-Alkyl, $C_5$-$C_{12}$-Cycloalkyl, Phenyl, o-, m- und p-Toluyl, o-, m- und p-Hydroxyphenyl, 3,5-Di-t-butyl-4-hydroxyphenyl, o-, m- und p-Methoxyphenyl, o-, m- und p-Aethoxyphenyl, Benzyl, 4-Methylbenzyl, 4-t-Butylbenzyl, 4-Hydroxybenzyl, 3,5-Di-t-butyl-4-hydroxybenzyl, 4-Methoxybenzyl, 4-Aethoxybenzyl oder eine Gruppe der Formel II bedeutet, oder Pyrrolidin-1-yl, Piperidin-1-yl, Morpholin-4-yl, Piperazin-1-yl, 4-Methyl-piperazin-1-yl, Hexahydroazepin-1-yl, Homopiperazin-1-yl oder 4-Methyl-homopiperazin-1-yl darstellt, oder mit y = 2 A einen Rest der Formel III

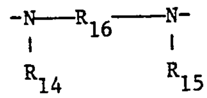

(III)

worin $R_{14}$ und $R_{15}$ gleich oder verschieden sein können und $C_5$-$C_{12}$-Cycloalkyl oder einen Rest der Formel II bedeuten und $R_{16}$ für $C_1$-$C_{20}$-Alkylen, $C_5$-$C_{12}$-Cycloalkylen, $C_6$-$C_{12}$-Arylen oder $C_7$-$C_{12}$-Aralkylen steht, oder einen Rest

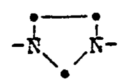 , 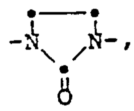 , 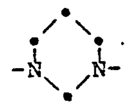 , 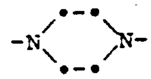 , 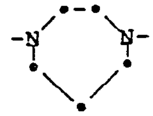 ,

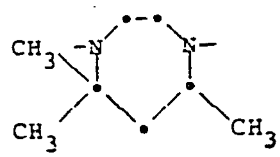 , 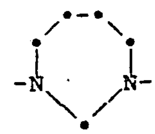 ;

darstellt, oder mit y = 3 A einen Rest der Formel IV

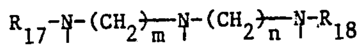

(IV)

worin $R_{17}$ und $R_{18}$ gleich oder verschieden sein können und $C_1$-$C_{12}$-Alkyl, $C_5$-$C_{12}$-Cycloalkyl oder einen Rest der Formel II bedeuten sowie m und n gleich oder verschieden sein können und ganze Zahlen von 2 bis 6 sind, oder einen Rest

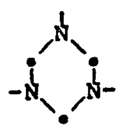 oder 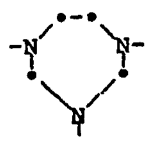

darstellt, oder mit y = 4, A einen Rest der Formel V

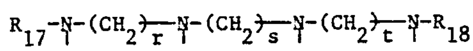

(V)

worin $R_{17}$ und $R_{18}$ die oben angegebenen Bedeutungen haben sowie r, s und t gleich oder verschieden sein können und ganze Zahlen von 2 bis 6 sind, oder einen Rest

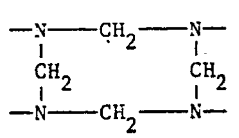

darstellt.

2. Verbindungen nach Anspruch 1, dadurch gekennzeichnet, dass $R_1$ für Wasserstoff, $C_1$-$C_6$-Alkyl, $C_3$-$C_6$-Alkenyl, Benzyl oder $C_1$-$C_6$-Acyl, $R_2$ und $R_6$ für Methyl, $R_4$ für Wasserstoff oder Methyl, $R_5$ für Wasserstoff, $R_3$ und $R_7$ für Methyl oder Aethyl, $R_{12}$ für einen Rest der Formel II, $R_{13}$ für Wasserstoff, $C_1$-$C_{12}$-Alkyl, $C_6$-$C_{12}$-Cycloalkyl, 2,2,6,6-Tetramethylpiperidin-4-yl oder 1,2,2,6,6-Pentamethylpiperidin-4-yl, $R_{14}$ und $R_{15}$ für $C_6$-$C_{12}$-Cycloalkyl, 2,2,6,6-Tetramethylpiperidin-4-yl oder 1,2,2,6,6-Pentamethylpiperidin-4-yl, $R_{16}$ für $C_1$-$C_6$-Alkylen, $R_{17}$ und $R_{18}$ für $C_1$-$C_8$-Alkyl, $C_6$-$C_{12}$-Cycloalkyl, 2,2,6,6-Tetramethylpiperidin-4-yl oder 1,2,2,6,6-Pentamethylpiperidin-4-yl, A, falls es einen heterocyclischen Rest darstellt, für Piperidin-1-yl, Morpholin-4-yl, Piperazin-1,4-diyl oder Hexahydro-1,3,5-triazin-1,3,5-triyl sowie m, n, r, s und t für 2 oder 3 stehen.

3. Verbindungen nach Anspruch 2, dadurch gekennzeichnet, dass $R_1$ für Wasserstoff, Methyl, Allyl, Benzyl oder Acetyl, $R_2$, $R_3$, $R_6$ und $R_7$ für Methyl, $R_4$ und $R_5$ für Wasserstoff, $R_{12}$ für 2,2,6,6-Tetramethylpiperidin-4-yl oder 1,2,2,6,6-Pentamethylpiperidin-4-yl, $R_{13}$ für Wasserstoff, $C_4$-$C_8$-Alkyl,

Cyclohexyl, 2,2,6,6-Tetramethylpiperidin-4-yl oder 1,2,2,6,6-Pentamethylpiperidin-4-yl, $R_{14}$, $R_{15}$, $R_{17}$ und $R_{18}$ für Cyclohexyl, 2,2,6,6-Tetramethylpiperidin-4-yl oder 1,2,2,6,6-Pentamethylpiperidin-4-yl und $R_{16}$ für $C_2$-$C_6$-Alkylen stehen.

4. Verbindungen nach Anspruch 1, dadurch gekennzeichnet, dass sie einer der folgenden Formeln entsprechen :

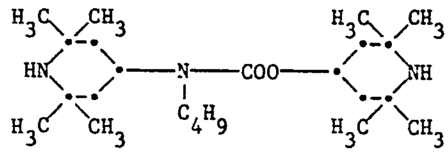

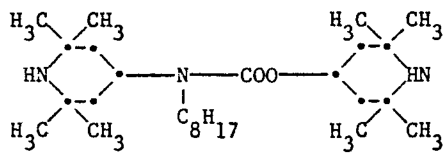

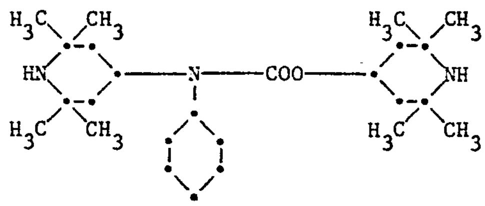

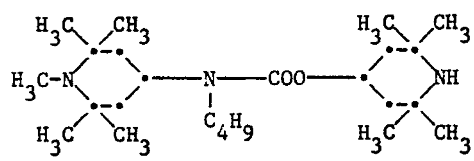

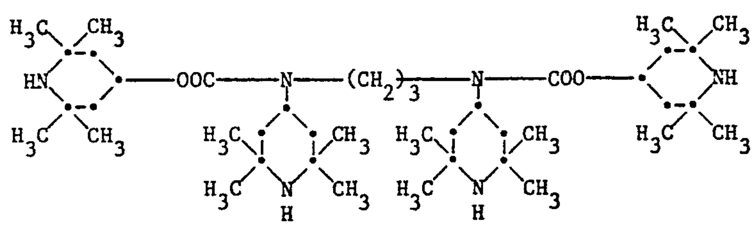

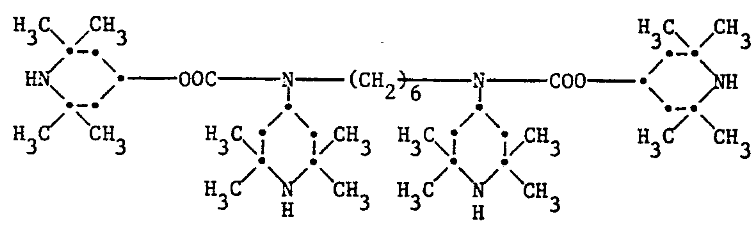

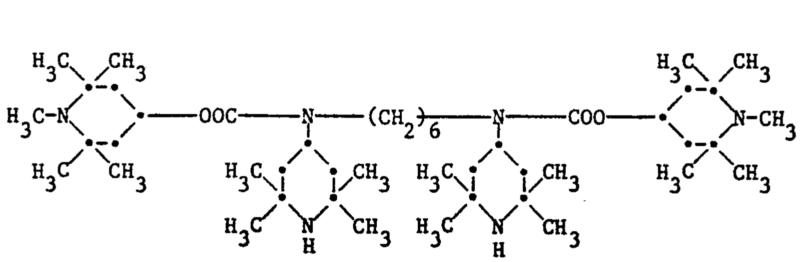

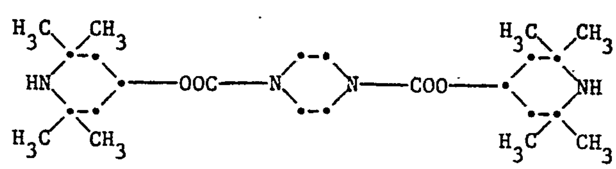

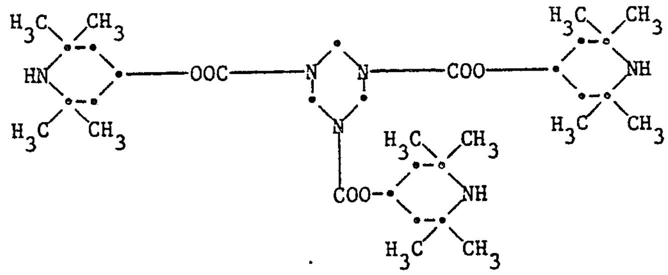

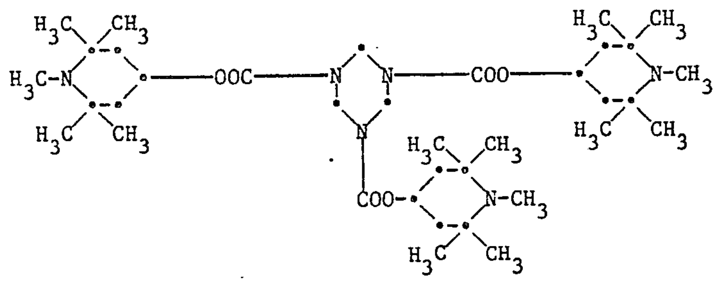

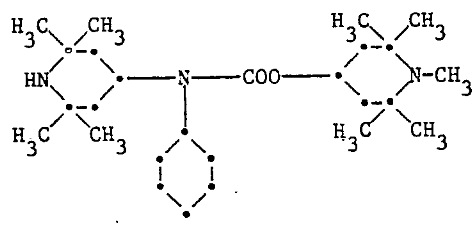

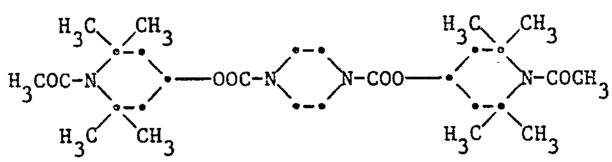

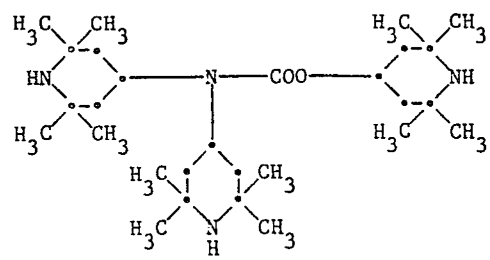

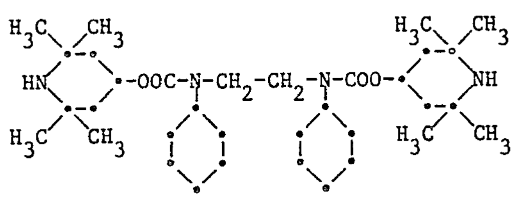

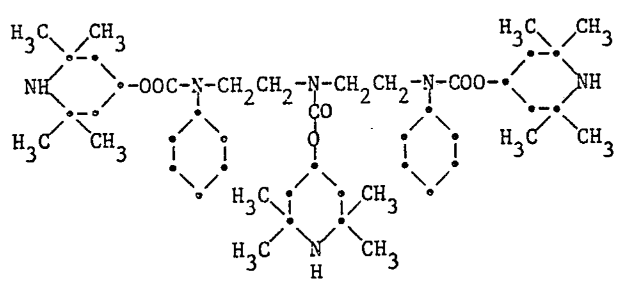

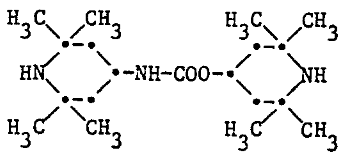

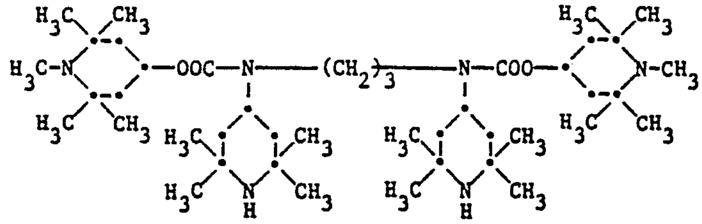

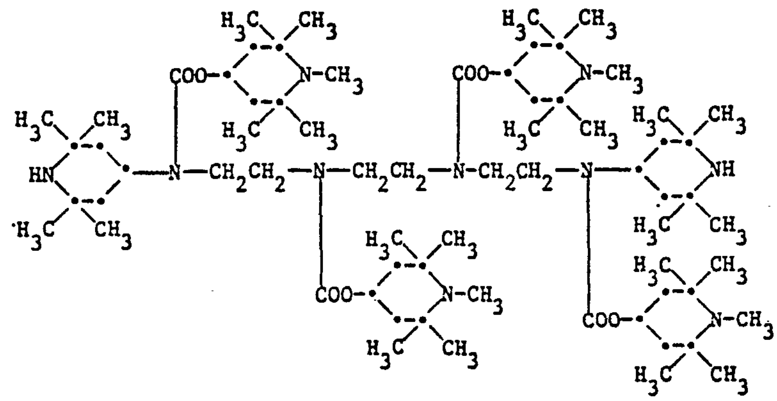

5. Verfahren zur Herstellung von Verbindungen der Formel I

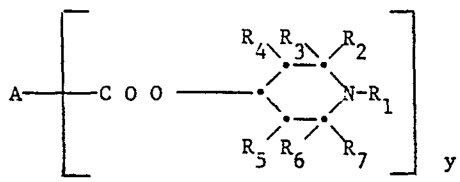

(I)

worin $R_1$, $R_2$, $R_3$, $R_4$, $R_5$, $R_6$, $R_7$, A und y die in Anspruch 1 angegebenen Bedeutungen haben, dadurch gekennzeichnet, dass man ein Piperidinol der Formel VI

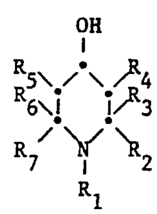

(VI)

in Gegenwart eines Umesterungskatalysators bei einer Temperatur zwischen 100 und 250 °C und einem Druck zwischen 0,1 und 760 Torr mit einem Ester der Formel VII

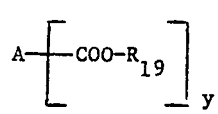

(VII)

worin $R_{19}$ für $C_1$-$C_4$-Alkyl oder Phenyl steht, umsetzt.

6. Verfahren nach Anspruch 5, dadurch gekennzeichnet, dass man den Umesterungskatalysator aus der Alkalialkoholate, $LiNH_2$, Aluminiumalkoholate, Tetraalkyltitanate, Dibutylzinnoxyd und $Sb_2O_3$ umfassenden Gruppe auswählt.

7. Verfahren zur Herstellung von Verbindungen der Formel I

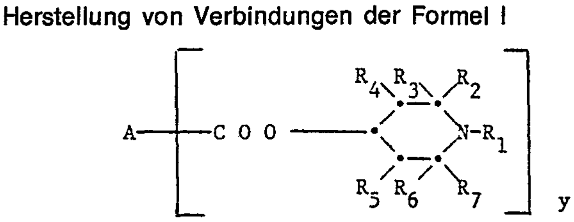

(I)

worin $R_1$, $R_2$, $R_3$, $R_4$, $R_5$, $R_6$, $R_7$, A und y die in Anspruch 1 angegebenen Bedeutungen haben, dadurch gekennzeichnet, dass man einen Chlorkohlensäureester der Formel VIII

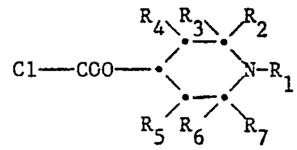

(VIII)

oder dessen Hydrochlorid in Gegenwart einer Base bei einer Temperatur zwischen − 30 und + 100 °C mit einem Amin der Formel IX

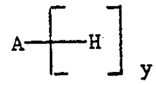

(IX)

umsetzt.

8. Verfahren nach Anspruch 7, dadurch gekennzeichnet, dass man die Base aus der Natrium- und Kalium-bicarbonat, -carbonat und -hydroxyd, Pyridin, Triäthylamin und Tributylamin umfassenden Gruppe auswählt.

9. Gegen Licht, Wärme und Oxydation stabilisierte Polymerzusammensetzungen, dadurch gekennzeichnet, dass sie aus einem synthetischen Polymeren und einer Stabilisatorverbindung der Formel I in einer Menge zwischen 0,01 und 5 Gew.-%, vorzugsweise zwischen 0,1 und 1 Gew.-%, bezogen auf das Gewicht des synthetischen Polymeren, bestehen.

10. Zusammensetzungen nach Anspruch 9, dadurch gekennzeichnet, dass sie zusätzlich zu dem neuen Stabilisator weitere übliche Zusatzstoffe für synthetische Polymere enthalten.

11. Zusammensetzungen nach Anspruch 9, dadurch gekennzeichnet, dass als synthetisches Polymer Polyäthylen oder Polypropylen vorliegt.

12. Aus nach Anspruch 9 stabilisierten Polymerzusammensetzungen erhaltene Erzeugnisse.

**Claims**

1. Compounds of the formula

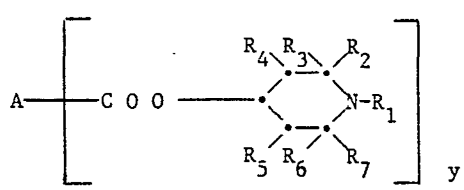

(I)

or acid addition salts thereof, in which $R_1$ represents hydrogen, $C_1$-$C_{12}$-alkyl, $—CH_2CN$, $C_3$-$C_{12}$-alkenyl or -alkinyl, benzyl, benzyl substituted by 1 to 3 $C_1$-$C_4$-alkyl radicals, hydroxybenzyl or hydroxybenzyl substituted by 1 to 3 $C_1$-$C_4$-alkyl radicals ; a $—COR_8$, $—COOR_8$, $—CH_2COOR_8$ or $—CONR_8R_9$ radical, in which $R_8$ and $R_9$, which may be identical or different, represent linear or branched $C_1$-$C_{12}$-alkyl, $C_3$-$C_{12}$-alkenyl, $C_5$-$C_{12}$-cycloalkyl, phenyl, phenyl substituted by 1 to 3 $C_1$-$C_8$-alkyl radicals, hydroxyphenyl, hydroxyphenyl substituted by 1 to 3 $C_1$-$C_8$-alkyl radicals, $C_7$-$C_{12}$-aralkyl, 2,2,6,6-tetramethyl-piperidin-4-yl or 1,2,2,6,6-pentamethyl-piperidin-4-yl or, when they are bonded to nitrogen, can be hydrogen or together with the N atom to which they are bonded can form a pyrrolidine, piperidine, morpholine, piperazine, N-methylpiperazine, homopiperazine or N-methylhomopiperazine ring ; or $R_1$ represents a

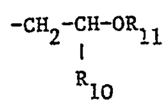

radical, in which $R_{10}$ is hydrogen, methyl or phenyl and $R_{11}$ is hydrogen, $—R_8$, $—COR_8$ or $—CONR_8R_9$, in which $R_8$ and $R_9$ are as defined above ; or $R_1$ represents a

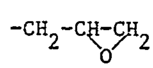

radical ; $R_2$, $R_3$, $R_6$ and $R_7$, which may be identical or different, are alkyl containing 1 to 6 C atoms ; $R_4$ and $R_5$, which may be identical or different, are hydrogen or alkyl containing 1 to 6 C atoms ; and y

represents an integer from 1 to 4 ; and if y = 1, A is a

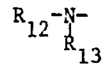

radical, in which $R_{12}$ is a radical of the formula II

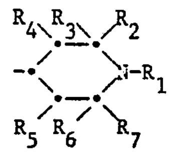 (II)

in which $R_1$, $R_2$, $R_3$, $R_4$, $R_5$, $R_6$ and $R_7$ have the above meanings ; $R_{13}$ is hydrogen, linear or branched $C_1$-$C_{20}$-alkyl, $C_5$-$C_{12}$-cycloalkyl, phenyl, o-, m- and p-toluyl, o-, m- and p-hydroxyphenyl, 3,5-di-t-butyl-4-hydroxyphenyl, o-, m- and p-methoxyphenyl, o-, m- and p-ethoxyphenyl, benzyl, 4-methybenzyl, 4-t-butylbenzyl, 4-hydroxybenzyl, 3,5-di-t-butyl-4-hydroxybenzyl, 4-methoxybenzyl, 4-ethoxybenzyl or a group of the formula II, or pyrrolidin-1-yl, piperidin-1-yl, morpholin-4-yl, piperazin-1-yl, 4-methylpiperazin-1-yl, hexahydro-azepin-1-yl, homopiperazin-1-yl or 4-methyl-homopiperazin-1-yl ; or if y = 2, A is a radical of the formula III

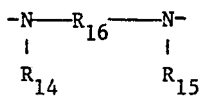 (III)

in which $R_{14}$ and $R_{15}$, which may be identical or different, are $C_5$-$C_{12}$-cycloalkyl, or a radical of the formula II, and $R_{16}$ is $C_1$-$C_{20}$-alkylene, $C_5$-$C_{12}$-cycloalkylene, $C_6$-$C_{12}$-arylene or $C_7$-$C_{12}$-aralkylene ; or A is a radical

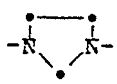 , 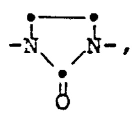 , 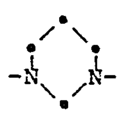 , 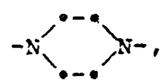 , 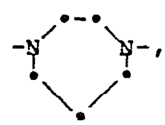 ,

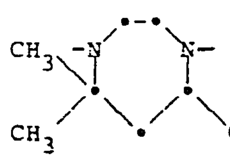 , 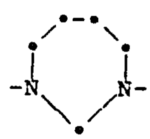 ;

or if y = 3, A is a radical of the formula IV

$$R_{17}-N-(CH_2)_m-N-(CH_2)_n-N-R_{18} \quad (IV)$$

in which $R_{17}$ and $R_{18}$ may be identical or different and represent $C_1$-$C_{12}$-alkyl, $C_5$-$C_{12}$-cycloalkyl or a radical of the formula II, and m and n, which may be identical or different, are integers from 2 to 6, or A is a radical

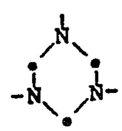 or 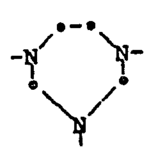 ;

or if y = 4, A is a radical of the formula V

$$R_{17}-N-(CH_2)_r-N-(CH_2)_s-N-(CH_2)_t-N-R_{18} \quad (V)$$

in which $R_{17}$ and $R_{18}$ have the abovementioned meanings and r, s and t, which may be identical or different, represent integers from 2 to 6, or A is a radical

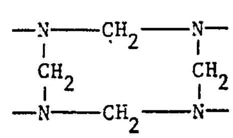

2. Compounds according to claim 1, characterised in that $R_1$ represents hydrogen, $C_1$-$C_6$-alkyl, $C_3$-$C_6$-alkenyl, benzyl or $C_1$-$C_6$-acyl ; $R_2$ and $R_6$ are methyl ; $R_4$ is hydrogen or methyl ; $R_5$ is hydrogen ; $R_3$ and $R_7$ are methyl or ethyl ; $R_{12}$ is a radical of formula II ; $R_{13}$ represents hydrogen, $C_1$-$C_{12}$-alkyl, $C_6$-$C_{12}$-cycloalkyl, 2,2,6,6-tetramethylpiperidin-4-yl or 1,2,2,6,6-pentamethylpiperidin-4-yl ; $R_{14}$ and $R_{15}$ represent $C_6$-$C_{12}$-cycloalkyl, 2,2,6,6-tetramethylpiperidin-4-yl or 1,2,2,6,6-pentamethylpiperidin-4-yl ; $R_{16}$ is $C_1$-$C_6$-alkylene ; $R_{17}$ and $R_{18}$ represent $C_1$-$C_8$-alkyl, $C_6$-$C_{12}$-cycloalkyl, 2,2,6,6-tetramethylpiperidin-4-yl or 1,2,2,6,6-pentamethylpiperidin-4-yl ; A, if it is a heterocyclic radical, is piperidin-1-yl, morpholin-4-yl, piperazin-1,4-diyl or hexahydro-1,3-5-triazin-1,3,5-triyl ; and m, n, r, s and t are 2 or 3.

3. Compounds according to claim 2, characterised in that $R_1$ is hydrogen, methyl, allyl, benzyl or acetyl ; $R_2$, $R_3$, $R_6$ and $R_7$ are methyl ; $R_4$ and $R_5$ are hydrogen ; $R_{12}$ is 2,2,6,6-tetramethylpiperidin-4-yl or 1,2,2,6,6-pentamethylpiperidin-4-yl ; $R_{13}$ is hydrogen, $C_4$-$C_8$-alkyl, cyclohexyl, 2,2,6,6-tetramethylpiperidin-4-yl or 1,2,2,6,6-pentamethylpiperidin-4-yl ; $R_{14}$, $R_{15}$, $R_{17}$ and $R_{18}$ are cyclohexyl, 2,2,6,6-tetramethylpiperidin-4-yl or 1,2,2,6,6-pentamethylpiperidin-4-yl ; and $R_{16}$ is $C_2$-$C_6$-alkylène.

4. Compounds according to claim 1, characterised in that they correspond to one of the formulae :

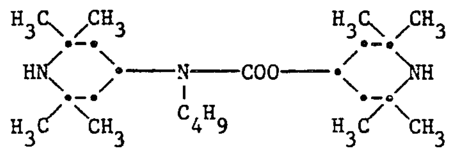

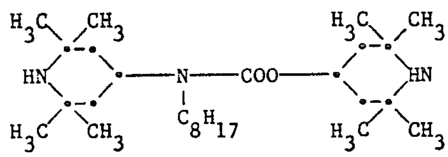

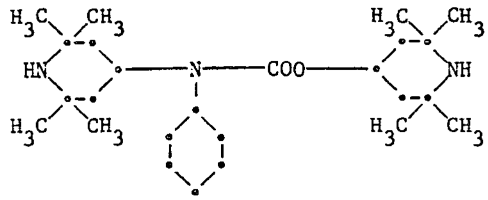

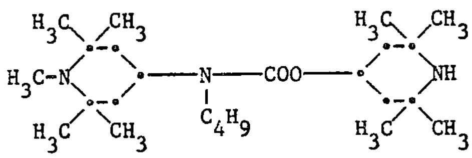

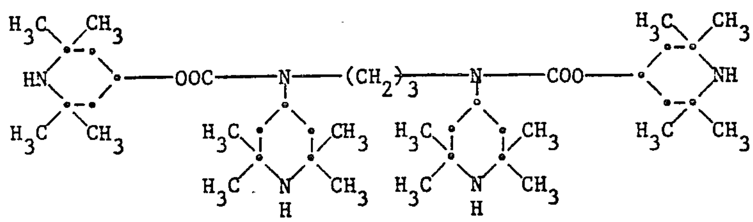

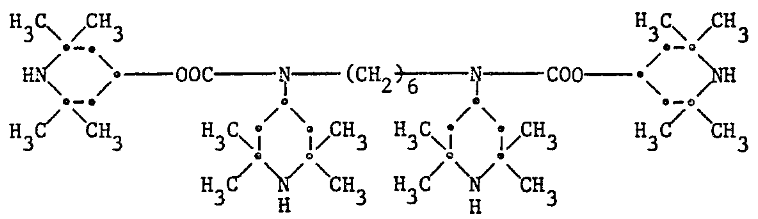

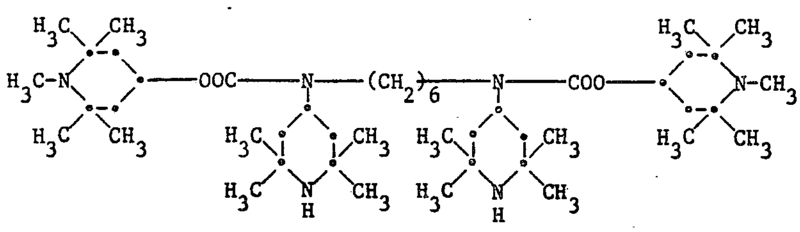

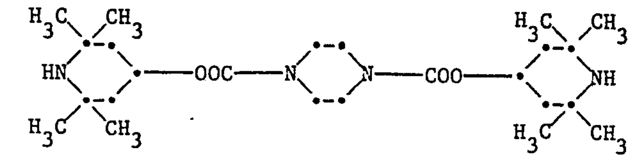

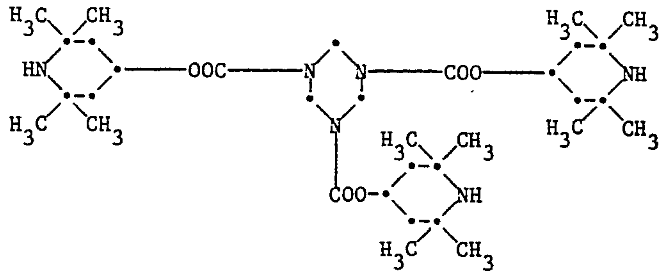

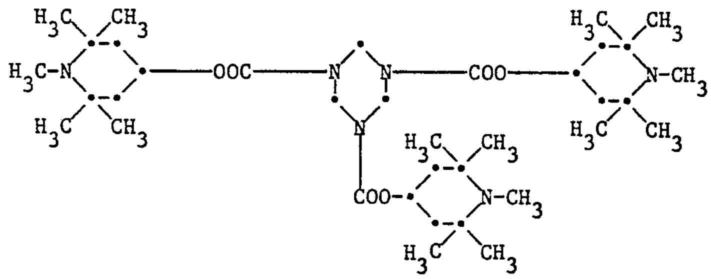

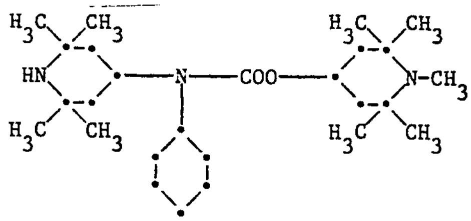

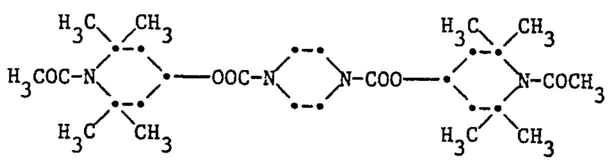

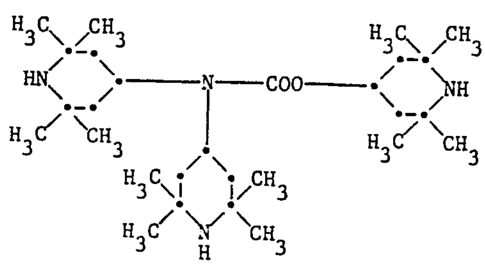

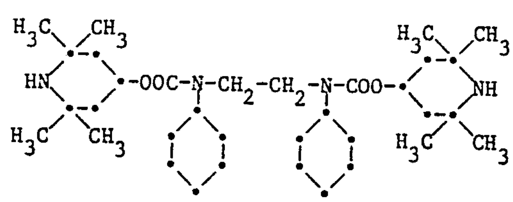

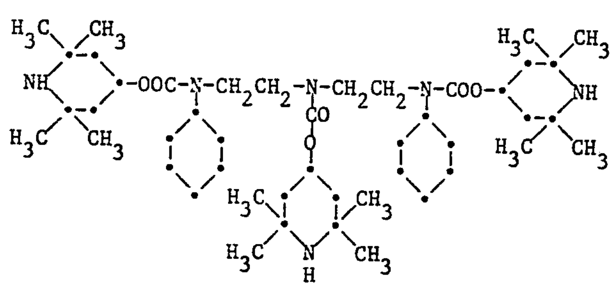

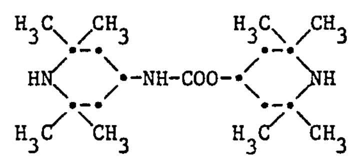

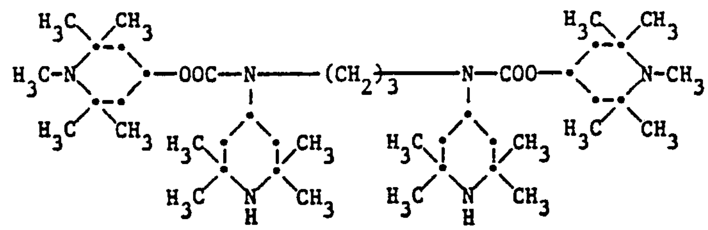

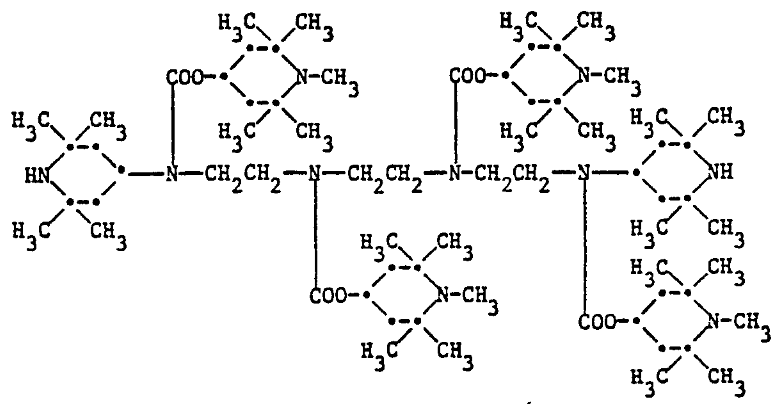

5. Process for the preparation of compounds of the formula I

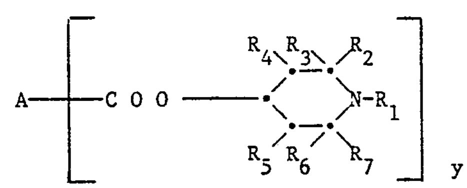

(I)

in which $R_1$, $R_2$, $R_3$, $R_4$, $R_5$, $R_6$, $R_7$, A and y are as defined in claim 1, characterised in that a piperidinol of the formula VI

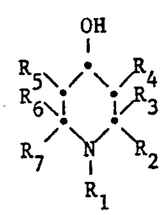

(VI)

is reacted with an ester of the formula VII

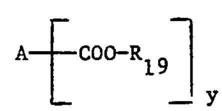

(VII)

in which $R_{19}$ is $C_1$-$C_4$-alkyl or phenyl, in the presence of a transesterification catalyst, at a temperature of between 100 and 250 °C and at a pressure of between 0.1 and 760 torr.

6. Process according to claim 5, characterised in that the transesterification catalyst is selected from the group comprising alkali metal alkoxides, $LiNH_2$, Al alkoxides, tetraalkyl titanates, dibutyl-tin oxide and $Sb_2O_3$.

7. Process for the preparation of compounds of the formula I

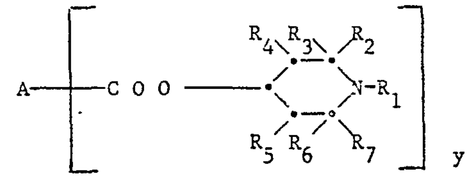

(I)

in which $R_1$, $R_2$, $R_3$, $R_4$, $R_5$, $R_6$, $R_7$, A and y are as defined in claim 1, characterised in that a chlorocarbonate of the formula VIII

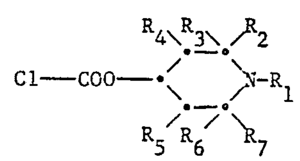

(VIII)

or the hydrochloride thereof is reacted with an amine of the formula IX

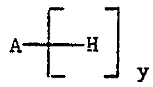

(IX)

in the presence of a base, at a temperature of between − 30° and + 100 °C.

8. Process according to claim 7, characterised in that the base is selected from the group comprising sodium bicarbonate, potassium bicarbonate, sodium carbonate, potassium carbonate, sodium hydroxide, potassium hydroxyde, pyridine, triethylamine and tributylamine.

9. Light-stabilised, heat-stabilised and oxidation-stabilised polymer compositions, characterised in that they consist of a synthetic polymer and a stabiliser compound of the formula I in an amount of between 0.01 and 5 %, preferably between 0.1 and 1 %, by weight, relative to the weight of the synthetic polymer.

10. Compositions according to claim 9, which, in addition to the novel stabiliser, contain other conventional additives for synthetic polymers.

11. Compositions according to claim 9, in which the synthetic polymer is polyethylene or polypropylene.

12. Articles obtained from stabilised polymer compositions according to claim 9.

## Revendications

1. Les composés de formule I

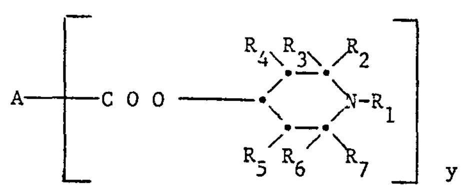

(I)

ainsi que leurs sels d'addition d'acides, formule dans laquelle $R_1$ représente l'hydrogène, un alkyle en $C_1$-$C_{12}$, un groupe —$CH_2CN$, un alcényle ou un alcynyle en $C_3$-$C_{12}$, un radical benzyle éventuellement substitué par 1 à 3 alkyles en $C_1$-$C_4$, un radical hydroxybenzyle éventuellement substitué par 1 à 3 alkyles en $C_1$-$C_4$, un radical —$COR_8$, —$COOR_8$, —$CH_2COOR_8$ ou —$CONR_8R_9$, $R_8$ et $R_9$, qui peuvent être identiques ou différents l'un de l'autre, étant des alkyles en $C_1$-$C_{12}$ ou des alcényles en $C_3$-$C_{12}$ linéaires ou ramifiés ou encore des cycloalkyles en $C_5$-$C_{12}$, un phényle éventuellement substitué par 1 à 3 alkyles en $C_1$-$C_8$, un hydroxyphényle éventuellement substitué par 1 à 3 alkyles en $C_1$-$C_8$, un aralkyle en $C_7$-$C_{12}$, un radical de 2,2,6,6-tétraméthyl-pipéridine-4-yle ou bien un radical 1,2,2,6,6-pentaméthyl-pipéridine-4-yle, ou, s'ils sont liés à l'azote, ils peuvent être l'hydrogène ou former, avec l'atome d'azote auquel ils sont liés, un radical pyrrolidinique, pipéridinique, morpholinique, pipérazinique, N-méthylpipérazinique, homopipérazinique ou N-méthylhomopipérazinique, ou encore un radical

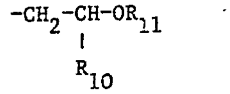

$R_{10}$ étant l'hydrogène, un méthyle ou un phényle et $R_{11}$ l'hydrogène ou un radical $—R_8$, $—COR_8$ ou $—CONR_8R_9$, $R_8$ et $R_9$ ayant les significations données ci-dessus, ou encore un radical

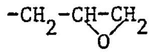

$R_2$, $R_3$, $R_6$ et $R_7$, qui peuvent être identiques ou différents les uns des autres, sont des alkyles en $C_1$ à $C_6$, $R_4$ et $R_5$, qui peuvent être identiques ou différents l'un de l'autre, représentent chacun l'hydrogène ou un alkyle en $C_1$ à $C_6$, et y est un entier de 1 à 4 ; et si y = 1, A représente un radical

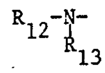

$R_{12}$ étant un radical de formule

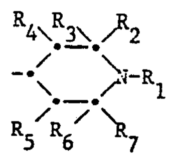
(II)

dans laquelle les divers symboles $R_1$, $R_2$, $R_3$, $R_4$, $R_5$, $R_6$ et $R_7$ ont les mêmes significations que ci-dessus, et $R_{13}$ étant l'hydrogène, un alkyle en $C_1$-$C_{20}$, linéaire ou ramifié, un cycloalkyle en $C_5$-$C_{12}$, un phényle, un o-, m- ou p-tpluyle, un o-, m- ou p-hydroxyphényle, un 3,5-di-t-butyl-4-hydroxyphényle, un o-, m- ou p-méthoxyphényle, un o-, m- et p-éthoxyphényle, un benzyle, un 4-méthylbenzyle, un 4-t-butylbenzyle, un 4-hydroxybenzyle, un 3,5-di-t-butyl-4-hydroxybenzyle, un 4-méthoxybenzyle, un 4-éthoxybenzyle ou un groupe de formule II, ou encore un radical pyrrolidine-1-yle, pipéridine-1-yle, morpholine-4-yle, pipérazine-1-yle, 4-méthyl-pipérazine-1-yle, hexahydrozépine-1-yle, homo-pipérazine-1-yle ou 4-méthyl-homopipérazine-1-yle ; si y = 2, A est un radical de formule III

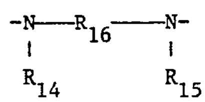
(III)

$R_{14}$ et $R_{15}$, qui peuvent être identiques ou différents l'un de l'autre, étant chacun un cycloalkyle en $C_5$-$C_{12}$ ou un groupe de formule II et $R_{16}$ représentant un alkylène en $C_1$-$C_{20}$, un cycloalkylène en $C_5$-$C_{12}$, un arylène en $C_6$-$C_{12}$ ou un aralkylène en $C_7$-$C_{12}$ ou encore un radical

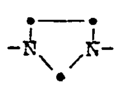 , 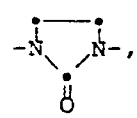 , 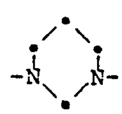 , 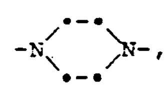 , 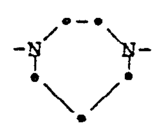 ,

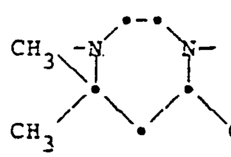 ou 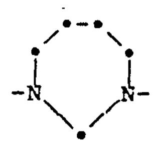 ;

si y = 3, A est un radical formule IV

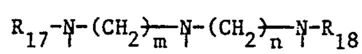
(IV)

dans laquelle $R_{17}$ et $R_{18}$, qui peuvent être identiques ou différents l'un de l'autre, sont chacun un alkyle en $C_1$-$C_{12}$, un cycloalkyle en $C_5$-$C_{12}$ ou un groupe de formule II, et m et n, identiques ou différents l'un de l'autre, sont des entiers de 2 à 6, ou encore un radical

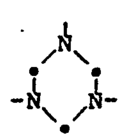 ou 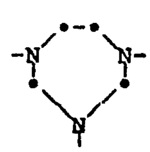 ;

ou bien si y = 4, A est un radical de formule

$$R_{17}-N-(CH_2)_r-N-(CH_2)_s-N-(CH_2)_t-N-R_{18} \qquad (V)$$

dans laquelle $R_{17}$ et $R_{18}$ ont les significations ci-dessus et r, s et t, qui peuvent être identiques ou différents les uns des autres, sont des entiers de 2 à 6, ou un radical

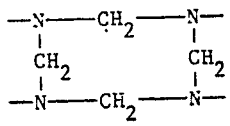

2. Composés selon la revendication 1 dans lesquels $R_1$ est l'hydrogène, un alkyle en $C_1$-$C_6$, un alcényle en $C_3$-$C_6$, un benzyle ou un acyle en $C_1$-$C_6$, $R_2$ et $R_6$ sont chacun un méthyle, $R_4$ est l'hydrogène ou un méthyle, $R_5$ l'hydrogène, $R_3$ et $R_7$ chacun un méthyle ou un éthyle, $R_{12}$ est un groupe de formule II, $R_{13}$ l'hydrogène, un alkyle en $C_1$-$C_{12}$, un cycloalkyle en $C_6$-$C_{12}$, ou bien un groupe 2,2,6,6-tétraméthylpipéridine-4-yle ou 1,2,2,6,6-pentaméthylpipéridine-4-yle, $R_{14}$ et $R_{15}$ sont chacun un cycloalkyle en $C_6$-$C_{12}$ ou bien un 2,2,6,6-tétraméthylpipéridine-4-yle ou 1,2,2,6,6-pentaméthylpipéridine-4-yle, $R_{16}$ est un alkylène en $C_1$-$C_6$, $R_{17}$ et $R_{18}$ sont chacun un alkyle en $C_1$-$C_8$, un cycloalkyle en $C_6$-$C_{12}$, un 2,2,6,6-tétraméthyl-pipéridine-4-yle ou un 1,2,2,6,6-pentaméthylpipéridine-4-yle, si A est un radical hétérocyclique, il s'agit d'un radical pipéridine-1-yle, morpholine-4-yle, pipérazine-1,4-diyle ou hexahydro-1,3,5-triazine-1,3,5-triyle, et m, n, r, s et t sont chacun le nombre 2 ou 3.

3. Composés selon la revendication 2 dans lesquels $R_1$ est l'hydrogène ou un groupe méthyle, allyle, benzyle ou acétyle, $R_2$, $R_3$, $R_6$ et $R_7$ sont chacun un méthyle, $R_4$ et $R_5$ l'hydrogène, $R_{12}$ un radical 2,2,6,6-tétraméthylpipéridine-4-yle ou 1,2,2,6,6-pentaméthyl-pipéridine-4-yle, $R_{13}$ l'hydrogène, un alkyle en $C_4$-$C_8$, un cyclohexyle, un 1,2,6,6-tétraméthylpipéridine-4-yle ou un 1,2,2,6,6-pentaméthylpipéridine-4-yle, $R_{14}$, $R_{15}$, $R_{17}$ et $R_{18}$ sont chacun un cyclohexyle, un 2,2,6,6-tétraméthylpipéridine-4-yle ou un 1,2,2,6,6-pentaméthylpipéridine-4-yle, et $R_{16}$ est un alkylène en $C_2$-$C_6$.

4. Composés selon la revendication 1, caractérisée en ce qu'ils répondent à l'une des formules suivantes :

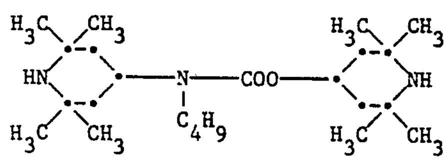

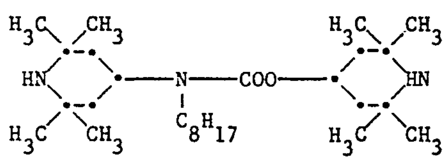

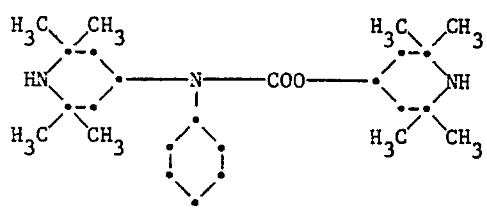

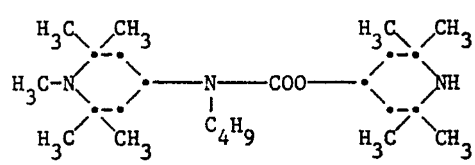

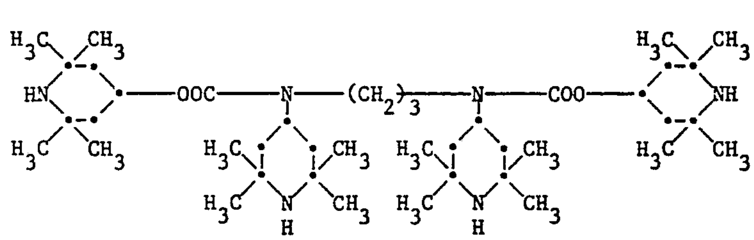

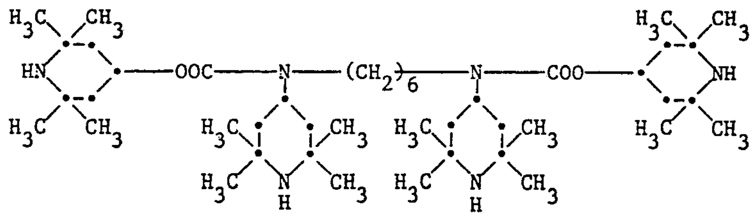

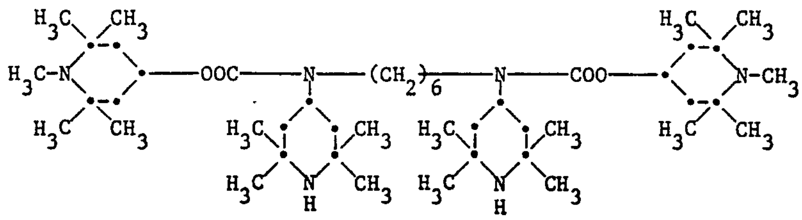

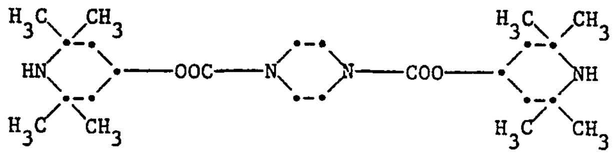

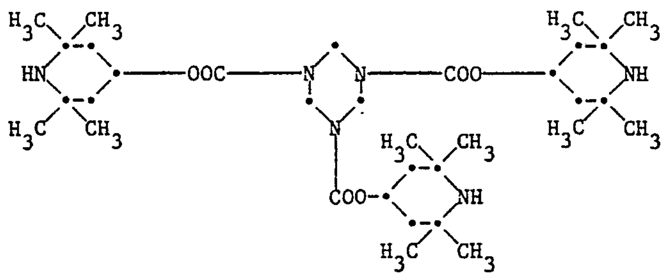

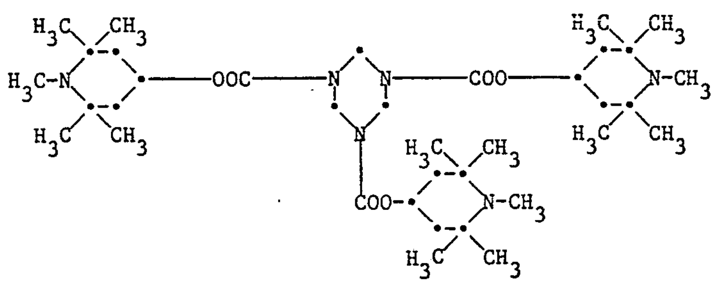

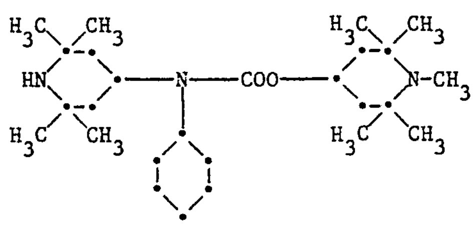

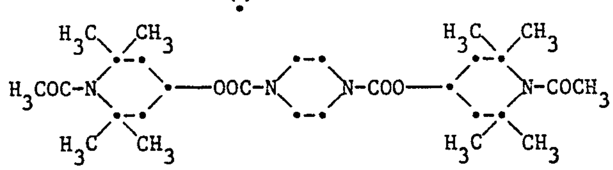

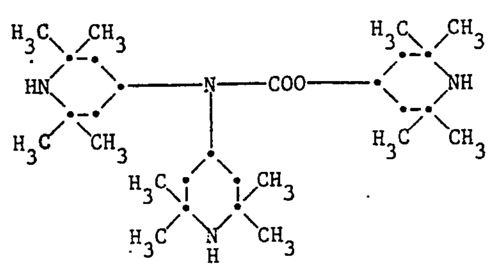

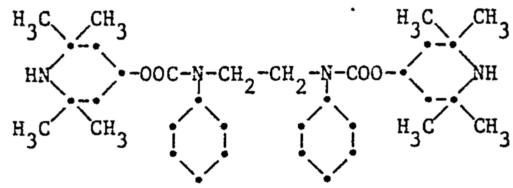

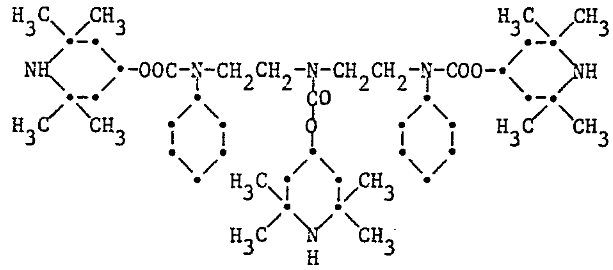

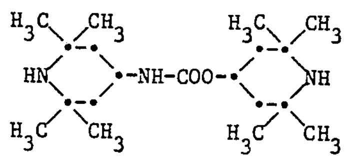

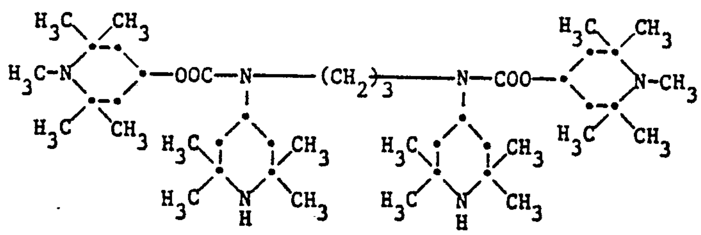

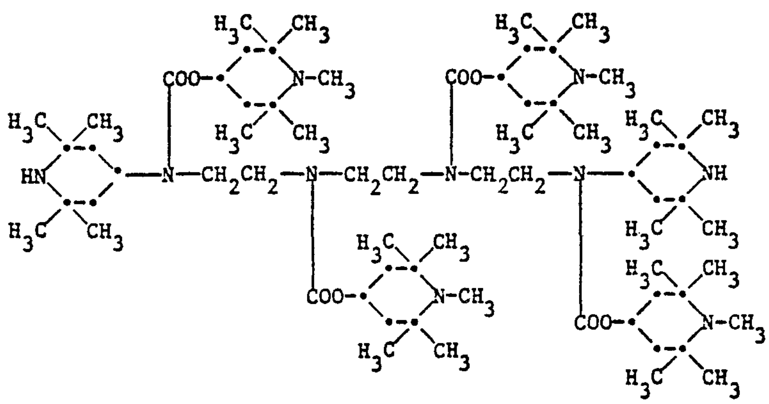

5. Procédé de préparation des composés de formule I selon la revendication 1, procédé caractérisé en ce qu'on fait réagir un pipéridinol de formule VI

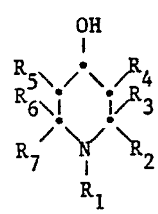

(VI)

en présence d'un catalyseur de transestérification, à une température de 100 à 250 °C sous une pression de 0,1 à 760 torr, avec un ester de formule VII

$$A\left[COO-R_{19}\right]_y \qquad (VII)$$

$R_{19}$ désignant un alkyle en $C_1$-$C_4$ ou un phényle.

6. Procédé selon la revendication 5 caractérisé en ce que le catalyseur de transestérification est choisi parmi des alcoolates de métaux alcalins, $LiNH_2$, des alcoolates d'aluminium, des titanates de tétraalkyles, l'oxyde de dibutyl-étain et $Sb_2O_3$.

7. Procédé de préparation des composés de formule I selon la revendication 1, caractérisé en ce que l'on fait réagir un chloroformiate de formule VIII

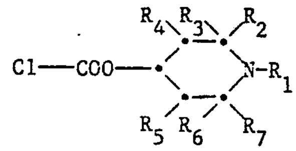

(VII)

ou son chlorhydrate en présence d'une base, à une température de − 30 à + 100 °C, avec une amine de formule IX

$$A\left[-H\right]_y \qquad \text{(IX)}$$

8. Procédé selon la revendication 7 caractérisé en ce que la base est choisi parmi le bicarbonate, le carbonate et l'hydroxyde de sodium ou de potassium, la pyridine, la triéthylamine et la tributylamine.

9. Compositions de matières polymères stabilisées contre l'action de la lumière, de chaleur et de l'oxydation, caractérisées en ce qu'elles comprennent un polymère synthétique avec un stabilisant de formule I selon la revendication 1 dans une proportion de 0,01 à 5 %, de préférence de 0,1 à 1 %, du poids du polymère.

10. Compositions selon la revendication 9 caractérisées en ce qu'elles contiennent en outre d'autres additifs usuels pour polymères synthétiques.

11. Compositions selon la revendication 9 ou 10, caractérisées en ce que le polymère est du polyéthylène ou du polypropylène.

12. Les produits finis qui ont été obtenus à partir de compositions de polymères stabilisées selon l'une quelconque des revendications 9 à 11.